# EUROPEAN PATENT APPLICATION

(11) **EP 2 689 847 A1**
(43) Date of publication of application: **29.01.2014**
(21) Application number: 12177407.9
(22) Date of filing: 22.07.2012
(51) Int. Cl.: B01J 31/00, C07F 15/00, C07H 21/00, C12Q 1/68

(54) **Methods for catalytic alkylation of nucleic acids**

(71) Applicant: Universität Basel, 4003 Basel (CH)
(72) Inventor: Gillingham, Dennis, 4056 Basel (CH); Tishinov, Kiril, 4056 Basel (CH)
(74) Representative: Kanopka, Martin

(57) **Abstract**

The present invention relates to methods and kits for catalytic alkylation of nucleic acids, and more particularly to methods and kits for selectively modifying nucleic acids by a rhodium catalyst and a substrate in a biological or an artificial composition.

SEQUENCE LISTING

<110>  UNIVERSITY OF BASEL

<120>  METHODS FOR CATALYTIC ALKYLATION OF NUCLEIC ACIDS

<130>  UNITEC01

<160>  27

<170>  PatentIn version 3.5

<210>  1
<211>  17
<212>  DNA
<213>  Homo sapiens

<400>  1
cgaacgtttt tcgttcg                                                      17


<210>  2
<211>  18
<212>  DNA
<213>  Homo sapiens

<400>  2
cgaacgtttt tcgttcga                                                     18


<210>  3
<211>  25
<212>  DNA
<213>  Homo sapiens

<400>  3
acggaattcc gtttttcgaa ttccg                                             25


<210>  4
<211>  17
<212>  DNA
<213>  Homo sapiens

<400>  4
cgaacgttat tcgttcg                                                      17


<210>  5
<211>  18
<212>  DNA
<213>  Homo sapiens

<400>  5
cuagcauuuu uugcuaga                                                     18


<210>  6
<211>  4
<212>  DNA
<213>  Homo sapiens

<400>  6
tttt                                                                     4

<210>  7
<211>  4
<212>  DNA
<213>  Homo sapiens

<400>  7
uuuu                                                                    4


<210>  8
<211>  4
<212>  DNA
<213>  Homo sapiens

<400>  8
aaaa                                                                    4


<210>  9
<211>  4
<212>  DNA
<213>  Homo sapiens

<400>  9
cccc                                                                    4


<210>  10
<211>  4
<212>  DNA
<213>  Homo sapiens

<400>  10
atgc                                                                    4


<210>  11
<211>  6
<212>  DNA
<213>  Homo sapiens

<400>  11
acugcu                                                                  6


<210>  12
<211>  6
<212>  DNA
<213>  Homo sapiens

<400>  12
actgct                                                                  6


<210>  13
<211>  6
<212>  DNA
<213>  Homo sapiens

<400>  13
ctctct                                                                  6


<210>  14

<211>  6
<212>  DNA
<213>  Homo sapiens

<400>  14
ctggct                                                                    6


<210>  15
<211>  15
<212>  DNA
<213>  Homo sapiens

<400>  15
tttatttgtt tcttt                                                         15


<210>  16
<211>  4
<212>  DNA
<213>  Homo sapiens

<400>  16
atgc                                                                      4


<210>  17
<211>  15
<212>  DNA
<213>  Homo sapiens

<400>  17
tttatttgtt tcttt                                                         15


<210>  18
<211>  4
<212>  DNA
<213>  Homo sapiens

<400>  18
atgc                                                                      4


<210>  19
<211>  4
<212>  DNA
<213>  Homo sapiens

<400>  19
atgc                                                                      4


<210>  20
<211>  4
<212>  DNA
<213>  Homo sapiens

<400>  20
atgc                                                                      4


<210>  21
<211>  4
<212>  DNA

<213>  Homo sapiens

<400>  21
atgc                                                                    4


<210>  22
<211>  15
<212>  DNA
<213>  Homo sapiens

<400>  22
tttatttgtt tcttt                                                       15


<210>  23
<211>  4
<212>  DNA
<213>  Homo sapiens

<400>  23
atgc                                                                    4


<210>  24
<211>  4
<212>  DNA
<213>  Homo sapiens

<400>  24
atgc                                                                    4


<210>  25
<211>  26
<212>  DNA
<213>  Homo sapiens

<400>  25
acggaattcc gtttttcgga attccg                                           26


<210>  26
<211>  15
<212>  DNA
<213>  Homo sapiens

<400>  26
tttatttgtt tcttt                                                       15


<210>  27
<211>  24
<212>  DNA
<213>  Homo sapiens

<400>  27
cccctaatac gactcactat aggg                                             24

## Description

### FIELD OF THE INVENTION

The present invention relates to novel methods and kits for catalytic alkylation of nucleic acids, and more particularly to novel methods and kits for selectively modifying nucleic acids by a rhodium catalyst and a substrate in a biological or an artificial composition. Further, the present invention relates to the modified nucleic acids in a biological or an artificial composition. In addition, the present invention relates to use of the said methods, nucleic acids, and/or kits.

### BACKGROUNG ART

Deciphering the complex puzzle of nucleic acid chemistry and biology has proven incredibly difficult and continues unabated after more than a century. Chemists have begun to recognize that the extraordinary informational and structural properties that have made nucleic acids so fundamental in biology could be exploited in other contexts. Therefore, chemical strategies to modify nucleic acids would have a significant impact on studying their varied functions as well as repurposing their special abilities.

Although there are various chemical methods to modify nucleic acids researchers typically resort to the effort of building a new phosphoramidite for solid-phase synthesis whenever an unnatural nucleic acid is required (for review see, e.g., Browne, K. A. Metal Ion-Catalyzed Nucleic Acid Alkylation and Fragmentation. Journal of the American Chemical Society 124, 7950-7962, (2002); Heetebrij, R. J. et al. Platinum (II)-Based Coordination Compounds as Nucleic Acid Labeling Reagents: Synthesis, Reactivity, and Applications in Hybridization Assays. ChemBioChem 4, 573-583, (2003); and Weisbrod, S. H. & Marx, A. Novel strategies for the site-specific covalent labeling of nucleic acids, Chemical Communications, 5675-5685 (2008).

In another method, the Cu-catalyzed azide-alkyne cycloaddition is used which allows a high degree of modularity from a single phosphoramidite (Rostovtsev, V. V., Green, L. G., Fokin, V. V. & Sharpless, K. B. A Stepwise Huisgen Cycloaddition Process: Copper(I)-Catalyzed Regioselective "Ligation" of Azides and Terminal Alkynes, Angewandte Chemie International Edition 41, 2596-2599, (2002); and Tomøe, C. W., Christensen, C. & Meldal, M. Peptidotriazoles on Solid Phase: [1,2,3]-Triazoles by Regiospecific Copper(I)-Catalyzed 1,3-Dipolar Cycloadditions of Terminal Alkynes to Azides. The Journal of Organic Chemistry 67, 3057-3064,(2002).

Other chemical methods to achieve selective internal alkylation employ a reactive molecule linked to a guiding motif to direct specificity (see for example, Zhou, Q. & Rokita, S. E. A general strategy for target-promoted alkylation in biological systems. Proceedings of the National Academy of Sciences 100, 15452-15457, (2003); Onizuka, K., Taniguchi, Y. & Sasaki, S. Site-Specific Covalent Modification of RNA Guided by Functionality-Transfer Oligodeoxynucleotides. Bioconjugate Chemistry 20, (2009); and Song, C.-X. & He, C. Bioorthogonal Labeling of 5-Hydroxymethylcytosine in Genomic DNA and Diazirine-Based DNA Photo-Cross-Linking Probes, Accounts of Chemical Research, null-null, (2011).

Further, an enzymatic method has been developed for NA modification which co-opts the natural S-adenosylmethionine (SAM)/methyltransferase system by employing SAM analogues bearing functional groups that can undergo subsequent chemoselective transformation (see, Dalhoff, C., Lukinavicius, G., Klimasauskas, S. & Weinhold, E. Direct transfer of extended groups from synthetic cofactors by DNA methyltransferases. Nat Chem Biol 2, 31-32, (2006); and Motorin, Y. et al. Expanding the chemical scope of RNA: methyltransferases to site-specific alkynylation of RNA for click labeling, Nucleic Acids Research 39, 1943-1952, (2011).

### DISCLOSURE OF THE INVENTION

### THE PROBLEMS AND THEIR SOLUTION

The major drawbacks the above-mentioned modification methods face is that they are unselective and/or labor-intensive. In addition, access to unnatural nucleic acids beyond the size limit of solid-phase synthesis is especially difficult. Furthermore, they require chemical manipulation of both the labeling group and the nucleic acid, in particular, the modification of nucleic acids occurs only after they have been altered chemically through solid-phase synthesis. Other modification methods involving a reactive molecule linked to a guiding motif to direct specificity are time-consuming, stoichiometric, and the guiding strand typically remains attached to the target after conjugation thus drastically changes nucleic acid properties. The enzymatic modification method is also complicated to establish. It can only accept one specialized structural motif (s-adenosylmethionine derivatives) and it is limited to those nucleic acid sequences and secondary structures that are naturally targeted by the enzyme. Therefore, it would be desirable to create alternative methods to achieve controlled modification of variety of nucleic acids in which modification occurs directly without the need of prior manipulation of the nucleic acid and without the need to synthesize complicated stoichiometric reagents for each new nucleic acid alkylation; are simple and user-friendly; avoid preparation of custom phosphoramidite; do not affect the ribophosphate backbone; are selective for exocyclic amine-containing nucleobases, in particular, are selective for unpaired nucleobases such as those present in single strands, mismatches, and bulge regions. The specificity of the method for unpaired nucleic acids sequences offers a new strategic tool for post-synthetic nucleic acid modification and may prove practical for chemical tailoring of DNA architectures in the burgeoning field of DNA nanotechnology (F. A. Aldaye, A. L. Palmer, H. F. Sleiman, Science 2008, 321, 1795-1799). In addition, it would be desirable to create alternative methods to achieve sequence specific alkylation that are simple and use the guiding motif in catalytic amounts. The greatest drawbacks of current techniques employing a guiding strand to direct specificity are: the need for stoichiometric amounts, the time-consuming synthesis, and the covalent attachment of the guiding strand to the target nucleic acid. Using a simple tethering technique to attach the guiding strand to a catalyst capable of alkylating nucleic acids would solve all of these problems. Ultimately, it is desirable to improve diagnosis, prognosis, and monitoring of cancer or other diseases associated with genetic abnormality in nucleic acids and to improve prognosis of unwanted drug side effects, for differentiating of cell types, tissue, or for examination of cell differentiation.

To solve the problems mentioned above, the present invention provides such methods to modify a nucleic acid in a biological or an artificial composition.

Other features and advantages of the invention will be apparent from the following description and from the claims.

### SUMMARY OF THE INVENTION

The present invention provides a method of selectively modifying a nucleic acid in a biological or an artificial composition, said method comprising the step of treating the composition with a rhodium catalyst and a substrate.

In further embodiment, the present invention provides a method of selectively modifying a nucleic acid in a biological or an artificial composition, said method comprising the step of treating the composition with a rhodium catalyst comprising a labile ligand set or a bidentate ligand set and a substrate. In further embodiment said substrate is a diazo substrate.

In further embodiment, the present invention provides a method of selectively modifying a nucleic acid in a biological or an artificial composition, said method comprising the step of treating the composition with a rhodium-carbenoid.

In another embodiment, the present invention provides a method of selectively modifying a nucleic acid in a biological or an artificial composition, said method comprising the step of treating the composition with a rhodium catalyst and a substrate, wherein said method comprising linking the rhodium catalyst to a guiding motif and wherein the guiding motif is identical, is complementary, or hybridizes under stringent or highly stringent conditions to all or portion of the nucleic acid. In further embodiment, said guiding motif comprising a functional group for association with the catalyst. In further embodiment, said ligand comprising a functional group for association to said guiding motif.

In another embodiment, the present invention provides a method of selectively modifying a nucleic acid in a biological or an artificial composition, said method comprising the step of treating the composition with a rhodium catalyst and a substrate, wherein the method further comprising the steps of (a) embedding the catalyst in a nucleic acid sequence or a nucleic acid mimic sequence, wherein the nucleic acid or the nucleic acid mimic is a stem-loop sequence, (b) inactivating the catalyst by a deactivating ligand at the opposite end of the said stem-loop sequence, (c) contacting the inactivated and embedded catalyst with the nucleic acid in a biological or an artificial composition, wherein the nucleic acid is identical, is complementary, or hybridizes under stringent conditions to all or portion of the stem-loop sequence;.

In another embodiment, the present invention provides a method of detecting a nucleic acid in a biological or an artificial composition, comprising (a) treating the composition comprising the nucleic acid with a rhodium catalyst and a substrate according to any of the above embodiments; (b) contacting the treated nucleic acid in (a) with a probe specific for said treated nucleic acid to form a complex comprising said treated nucleic acid and said probe hybridized to said treated nucleic acid, and (c) detecting hybridization of said probe to said nucleic acid.

In another embodiment, the present invention provides a method of detecting a nucleic acid in a biological or an artificial composition, further to the above embodiment comprising (a) embedding the catalyst in a nucleic acid sequence or a nucleic acid mimic sequence, wherein the nucleic acid is a stem-loop sequence, (b) inactivating the catalyst by a deactivating ligand at the opposite end of the said stem-loop sequence, (c) contacting the inactivated and embedded catalyst with the nucleic acid in a biological or an artificial composition, wherein the nucleic acid is identical, is complementary, or hybridizes under stringent or highly stringent conditions to all or portion of the stem-loop sequence, (d) contacting the modified nucleic acid in (c) with a probe specific for said modified nucleic acid to form a complex comprising said modified nucleic acid and said probe hybridized to said modified nucleic acid, and (e) detecting hybridization of said probe to said nucleic acid.

In another embodiment, the present invention relates to an oligonucleotide for amplifying a nucleic acid modified according to any of the proceeding embodiments, which is identical, is complementary, or hybridizes under stringent or highly stringent conditions to a nucleic acid. In further embodiment, the present invention relates to an oligonucleotide set for amplifying a nucleic acid, wherein the oligonucleotide set comprises a pair of oligonucleotides, and at least one of the pair of oligonucleotide is an oligonucleotide according to above the above-mentioned embodiment.

In another embodiment, the present invention provides a method according to any of the proceeding embodiments further comprising amplifying a nucleic acid sequence contained in a biological or an artificial composition, comprising (a) adding an oligonucleotide according to the above embodiments to the biological or an artificial composition, (b) amplifying the nucleic acid contained in the biological or an artificial sample using the oligonucleotide according to the above oligonucleotide embodiments. In further embodiment, said nucleic acid detection and amplification of the present invention is carried out in the presence of a mismatched and/or unpaired nucleobase and/or series of mismatched and/or unpaired nucleobases.

In another embodiment, the present invention provides a method of determining the presence or absence of a genetic abnormality in a nucleic acid contained in a biological or an artificial composition, comprising (a) amplifying the nucleic acid contained in a biological or an artificial composition by a nucleic acid amplification method according to the above embodiments, (b) measuring a fluorescence intensity before and after amplifying the target nucleic acid sequence in (a), and, (c) detecting the presence or absence of a genetic abnormality by comparing the fluorescence intensities measured in (b). In further embodiment, the present invention provides a method of determining the presence or absence of a genetic abnormality in a nucleic acid contained in a biological or an artificial composition, wherein the genetic abnormality comprises a mismatched nucleobase, series of a mismatched nucleobases, an unpaired nucleobase, a series of unpaired nucleobases, or a series of repeated nucleobases expansions.

In another embodiment, the present invention provides a nucleic acid modified according to any of the proceeding embodiments, and sequences complementary thereto. In further embodiment a nucleic acid modified according to the proceeding embodiments is Short-hairpin RNA, double-stranded RNA, or microRNAs with 3'-overhangs.

In another embodiment, the present invention provides a kit for selectively modifying a nucleic acid in a biological or an artificial composition, wherein the kit comprises (a) a rhodium catalyst (b) a substrate (c) containers suitable for containing the said rhodium catalyst, a substrate, and the biological or the artificial composition. In further embodiment, the present invention provides a kit wherein said catalyst further comprising a guiding motif, wherein the guiding motif is identical, is complementary, or hybridizes under stringent or highly stringent conditions to all or portion of the nucleic acid. In further embodiment, said guiding motif comprising a functional group for association with the catalyst. In further embodiment, the catalyst comprising a ligand that contains a functional group that facilitates association to said guiding motif. In another embodiment, the present invention provides a kit for detecting and/or amplifying a nucleic acid in a biological or an artificial composition, wherein the kit comprising (a) a rhodium catalyst (b) a substrate (c) an oligonucleotide, or an oligonucleotide set.

Additional embodiments provide use of the methods, nucleic acids, oligonucleotides and/or kits for modifying, amplifying and detecting a nucleic acid in a biological or an artificial composition, for alkylating a RNA *in vivo* to alter protein production, RNA interference, and/or signaling between one or series of RNA, for generating chemical libraries of modified RNA for use in controlling or modulating RNA interference, or diseases of RNA misregulation, for detecting genetic abnormality in a nucleic acid, for diagnosis, prognosis, monitoring and/or classification of cellular proliferative disorders, and for prognosis of unwanted drug side effects.

### DRAWINGS

Those of skill in the art will understand that the drawings, described below, are for illustrative purposes only. The drawings are not intended to limit the scope of the present teachings in any way.
Figure 1A. Illustrates proof of concept for rhodium-catalyzed NA alkylation.
Figure 1B. Illustrates proof of concept for rhodium-catalyzed NA alkylation targeting unpaired bases. A variety of nucleic acids can be catalytically alkylated with rhodium-carbenoids generated from diazo compounds in aqueous buffer through an N-H insertion process. The method specifically targets unpaired bases such as those present in single strands, turn regions, and overhangs while leaving double-stranded sequences untouched.
Figure 2. Depicts application of the catalytic alkylation in PCR labeling.
Figure 3. Illustrates a catalyst linked to a guiding motif for modifying a nucleic acid.
Figure 4. depicts HPLC-MS/MS analysis of the modification reaction of d(ATGC) with Dz-1 (HPLC analysis by method A).
Figure 5. HPLC trace of the modification reaction of d(A₄) with Dz-1 (HPLC analysis by method A)
Figure 6. HPLC trace of the modification reaction of d(C₄) with Dz-1 (HPLC analysis by method A)
Figure 7. HPLC trace of the modification reaction of a mixture of d(A₄), d(G₄), d(C₄) and d(T₄) with Dz-1 (HPLC analysis by method A)
Figure 8. HPLC trace of the modification reaction of a mixture of d(TAT) with Dz-1 (HPLC analysis by method A)
Figure 9. MS/MS Fragmentation of the singly modified d(TAT) with Dz-1
Figure 10. MS³ Fragmentation of the singly modified d(TAT) with Dz-1 - MS/MS fragmentation of the modified base (adenine)
Figure 11. HPLC trace of the modification reaction of a mixture of d(TCT) with Dz-1 (HPLC analysis by method A)
Figure 12. MS/MS Fragmentation of the singly modified d(TCT) with Dz-1
Figure 13. MS³ Fragmentation of the singly modified d(TCT) with Dz-1 - MS/MS fragmentation of the modified base (cytosine)
Figure 14. MS/MS Fragmentation of the singly modified d(TGT) with Dz-1

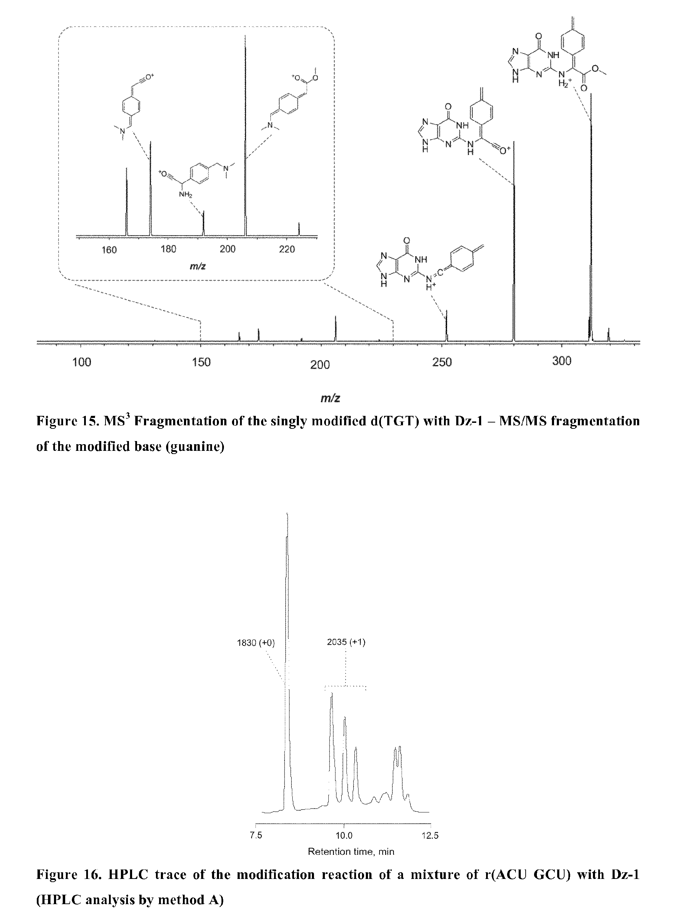

Figure 15. MS³ Fragmentation of the singly modified d(TGT) with Dz-1 - MS/MS fragmentation of the modified base (guanine)
Figure 16. HPLC trace of the modification reaction of a mixture of r(ACU GCU) with Dz-1 (HPLC analysis by method A)

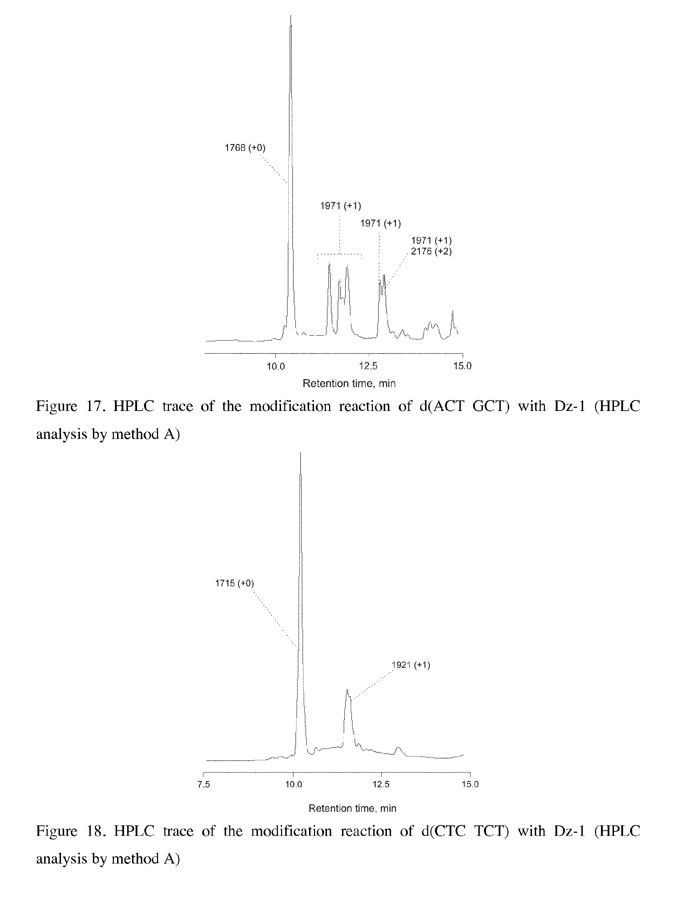

Figure 17. HPLC trace of the modification reaction of d(ACT GCT) with Dz-1 (HPLC analysis by method A)
Figure 18. HPLC trace of the modification reaction of d(CTC TCT) with Dz-1 (HPLC analysis by method A)

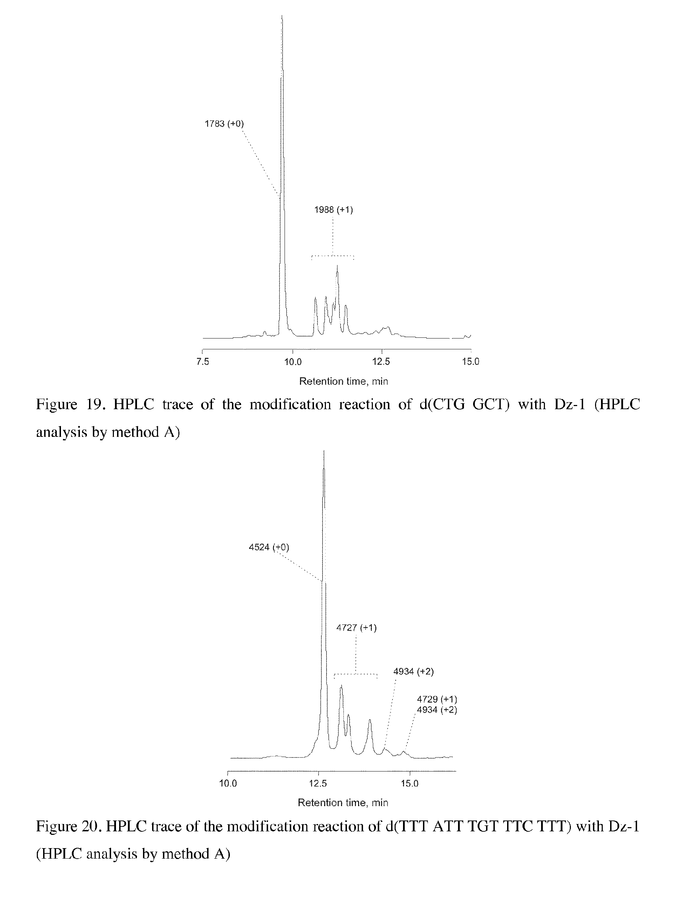

Figure 19. HPLC trace of the modification reaction of d(CTG GCT) with Dz-1 (HPLC analysis by method A)
Figure 20. HPLC trace of the modification reaction of d(TTT ATT TGT TTC TTT) with Dz-1 (HPLC analysis by method A)

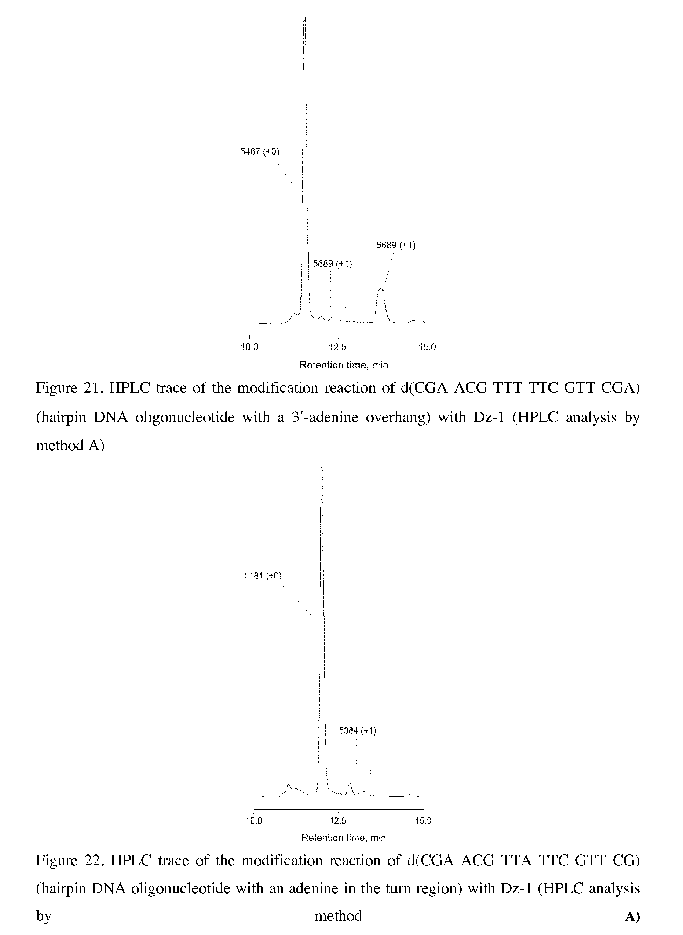

Figure 21. HPLC trace of the modification reaction of d(CGA ACG TTT TTC GTT CGA) (hairpin DNA oligonucleotide with a 3'-adenine overhang) with Dz-1 (HPLC analysis by method A)
Figure 22. HPLC trace of the modification reaction of d(CGA ACG TTA TTC GTT CG) (hairpin DNA oligonucleotide with an adenine in the turn region) with Dz-1 (HPLC analysis by method A)

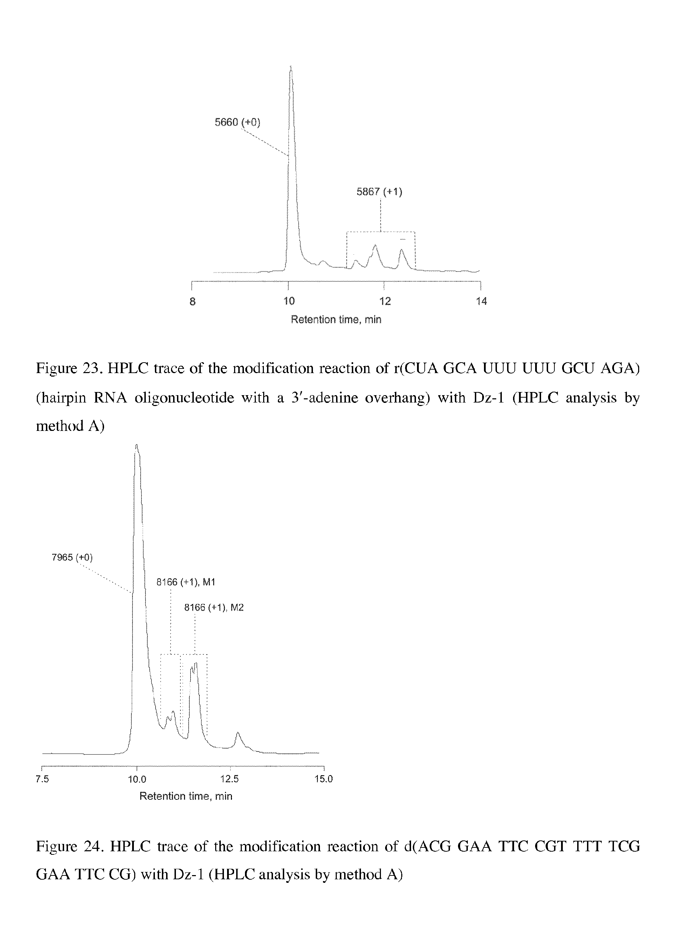

Figure 23. HPLC trace of the modification reaction of r(CUA GCA UUU UUU GCU AGA) (hairpin RNA oligonucleotide with a 3'-adenine overhang) with Dz-1 (HPLC analysis by method A)
Figure 24. HPLC trace of the modification reaction of d(ACG GAA TTC CGT TTT TCG GAA TTC CG) with Dz-1 (HPLC analysis by method A)

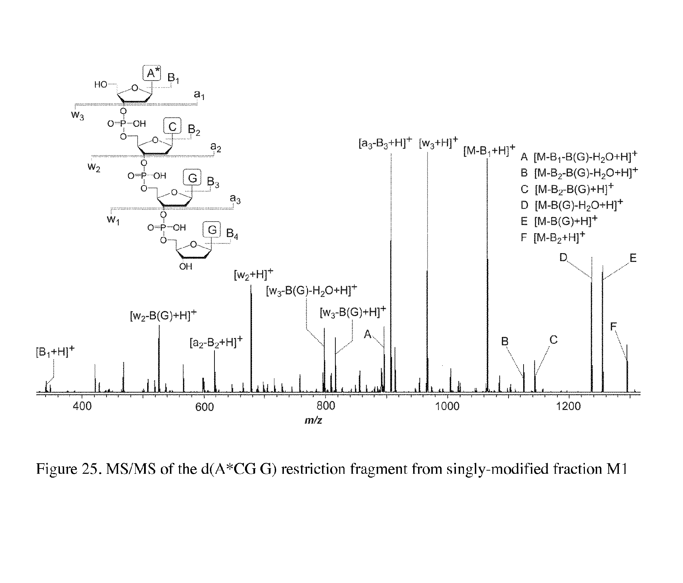

Figure 25. MS/MS of the d(A*CG G) restriction fragment from singly-modified fraction M1

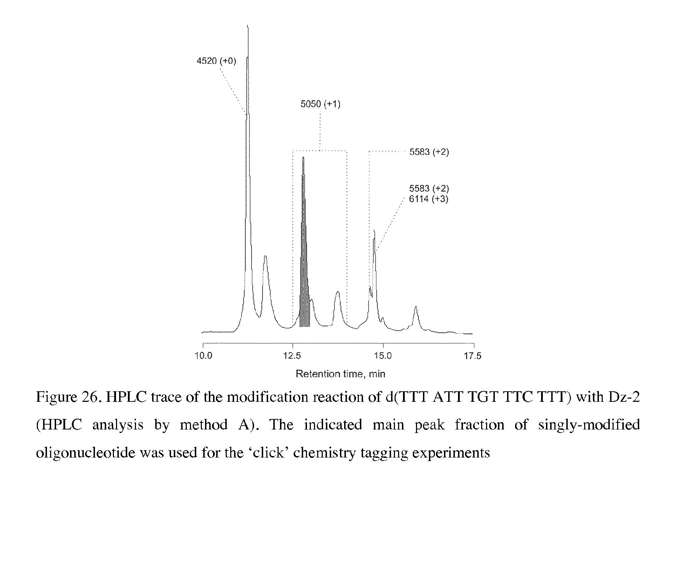

Figure 26. HPLC trace of the modification reaction of d(TTT ATT TGT TTC TTT) with Dz-2 (HPLC analysis by method A). The indicated main peak fraction of singly-modified oligonucleotide was used for the 'click' chemistry tagging experiments

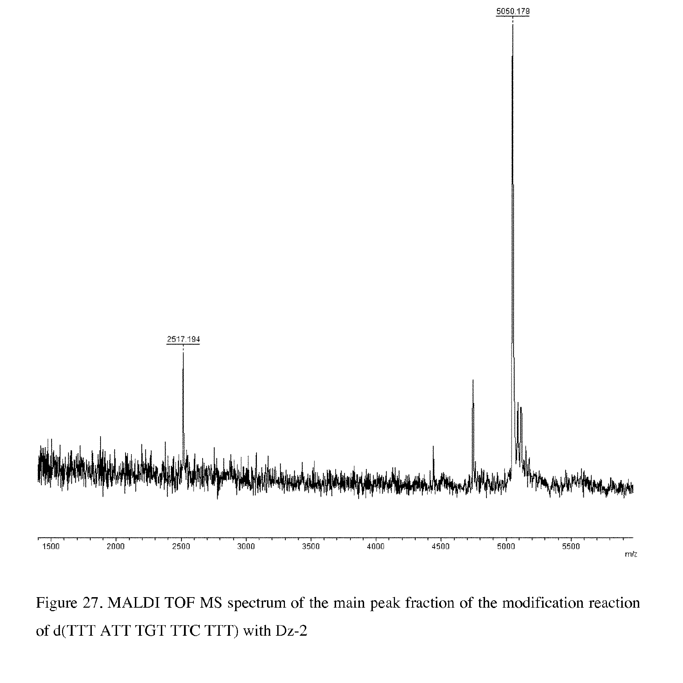

Figure 27. MALDI TOF MS spectrum of the main peak fraction of the modification reaction of d(TTT ATT TGT TTC TTT) with Dz-2

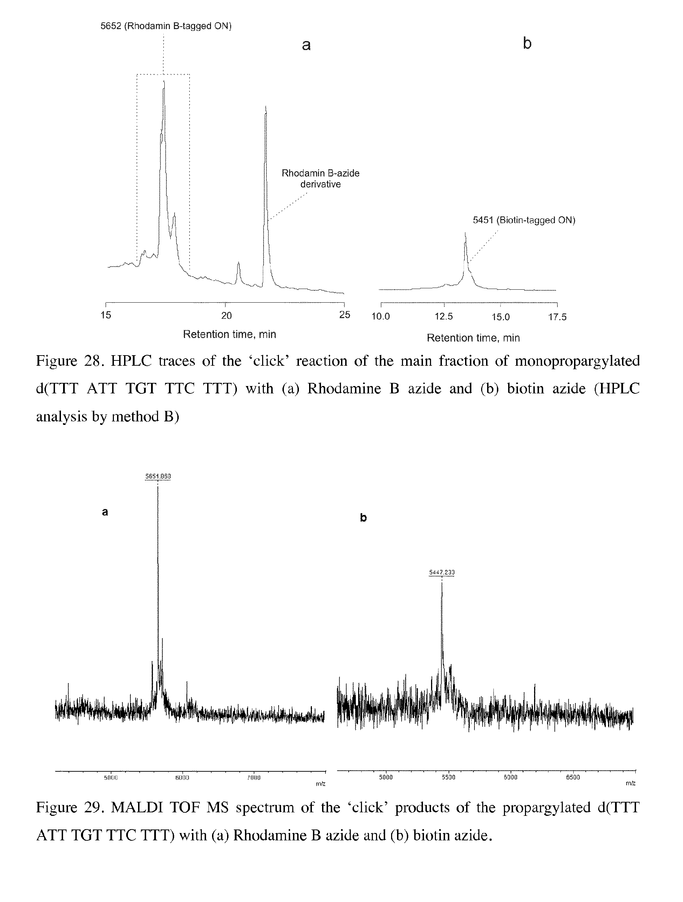

Figure 28. HPLC traces of the 'click' reaction of the main fraction of monopropargylated d(TTT ATT TGT TTC TTT) with (a) Rhodamine B azide and (b) biotin azide (HPLC analysis by method B)
Figure 29. MALDI TOF MS spectrum of the 'click' products of the propargylated d(TTT ATT TGT TTC TTT) with (a) Rhodamine B azide and (b) biotin azide.

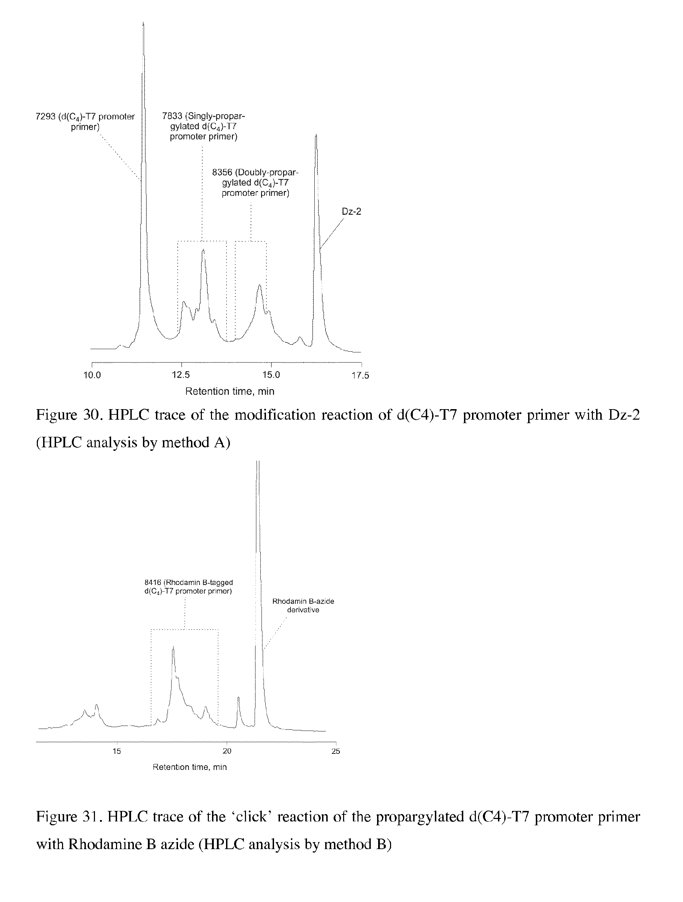

Figure 30. HPLC trace of the modification reaction of d(C4)-T7 promoter primer with Dz-2 (HPLC analysis by method A)
Figure 31. HPLC trace of the 'click' reaction of the propargylated d(C4)-T7 promoter primer with Rhodamine B azide (HPLC analysis by method B)

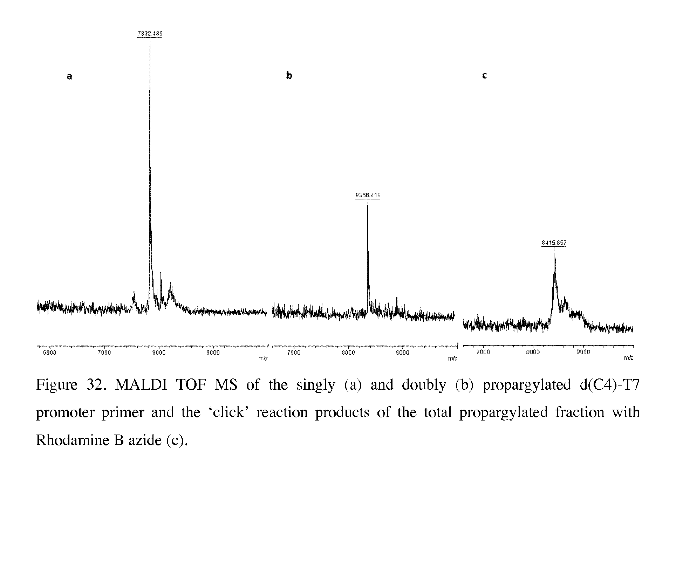

Figure 32. MALDI TOF MS of the singly (a) and doubly (b) propargylated d(C4)-T7 promoter primer and the 'click' reaction products of the total propargylated fraction with Rhodamine B azide (c)
Figure 33A. Illustrates synthetic sequence for preparation of Dz-1 (3).
Figure 33B. Illustrates synthetic sequence for preparation of Dz-2 (4).

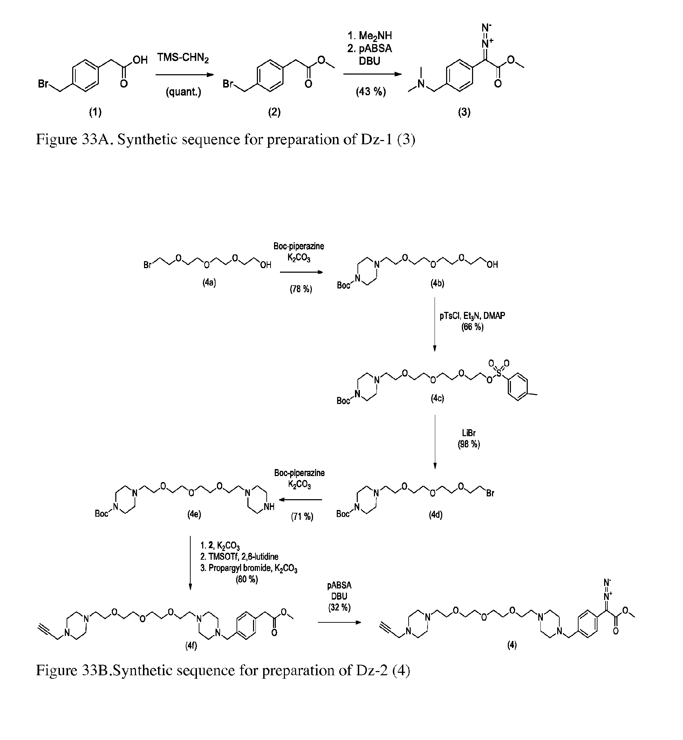

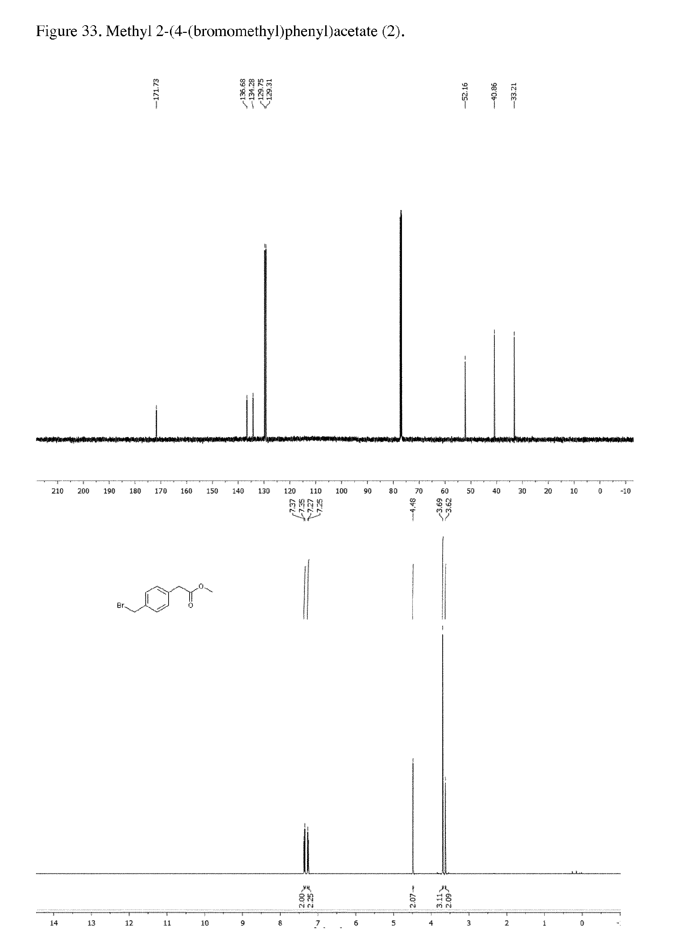

Figure 33. Shows analytical TLC, ¹H and ¹³C NMR spectra, EI MS spectra of Methyl 2-(4-(bromomethyl)phenyl)acetate (2).

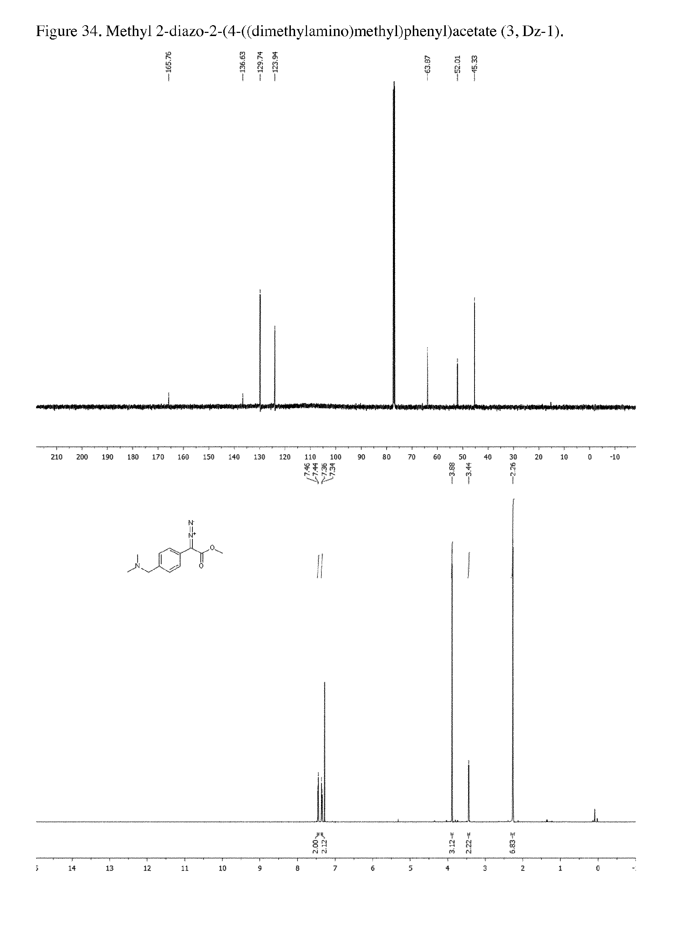

Figure 34. Shows analytical TLC, ¹H and ¹³C NMR spectra, ESI MS spectra of Methyl 2-diazo-2-(4-((dimethylamino)methyl)phenyl)acetate (3, Dz-1).

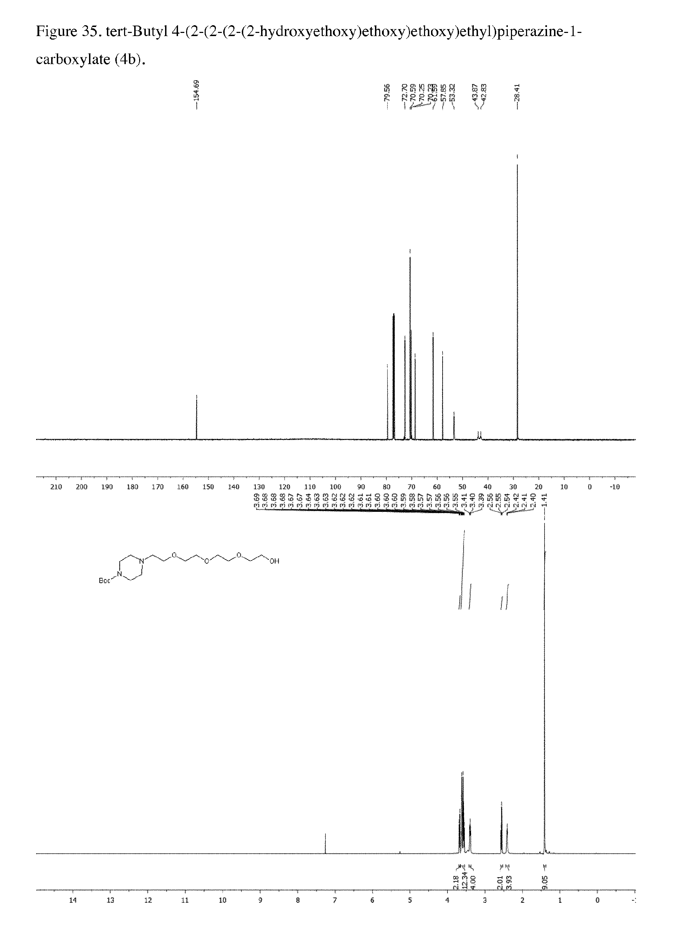

Figure 35. Shows analytical TLC, ¹H and ¹³C NMR spectra, ESI MS spectra of tert-Butyl 4-(2-(2-(2-(2-hydroxyethoxy)ethoxy)ethoxy)ethyl)piperazine-1-carboxylate (4b).

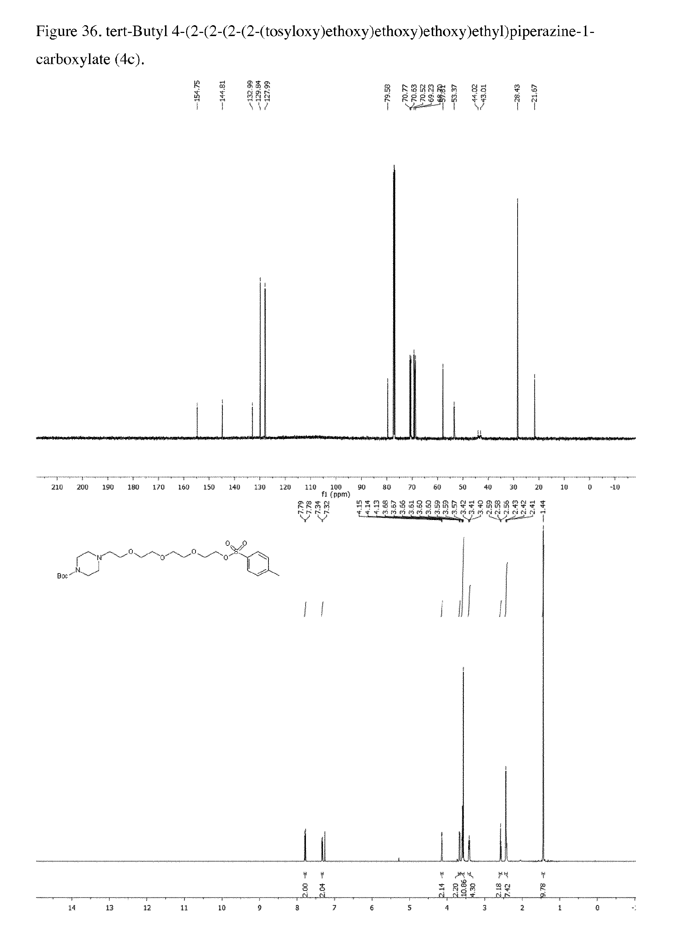

Figure 36. Shows analytical TLC, ¹H and ¹³C NMR spectra, ESI MS spectra of tert-Butyl4-(2-(2-(2-(2-(tosyloxy)ethoxy)ethoxy)ethoxy)ethyl)piperazine-1-carboxylate (4c).

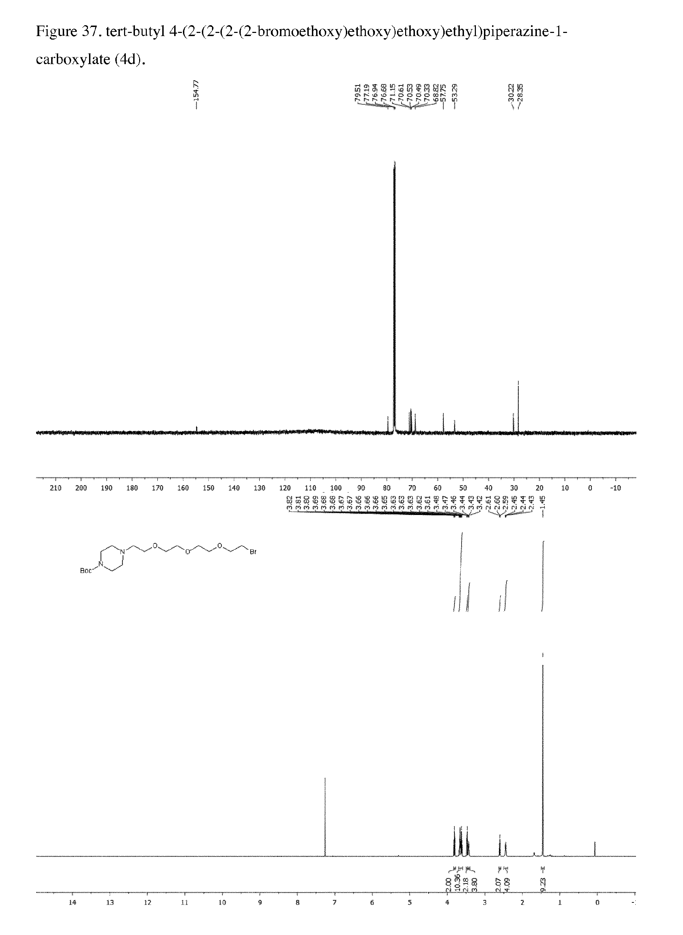

Figure 37. Shows analytical TLC, ¹H and ¹³C NMR spectra, ESI MS spectra of tert-butyl 4-(2-(2-(2-(2-bromoethoxy)ethoxy)ethoxy)ethyl)piperazine-1-carboxylate (4d).

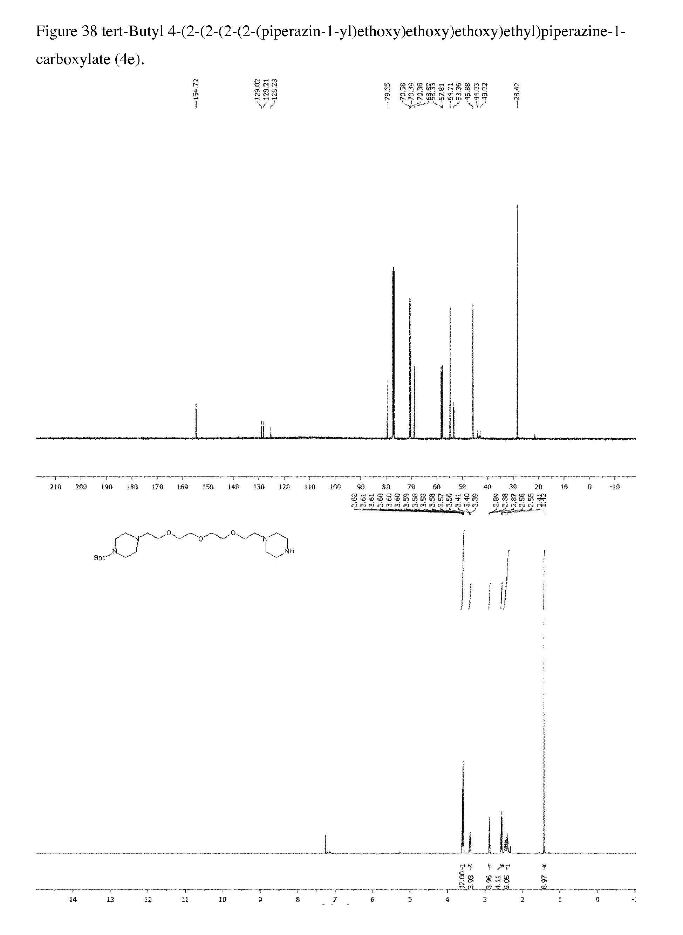

Figure 38. Shows analytical TLC, ¹H and ¹³C NMR spectra, ESI MS spectra of tert-Butyl4-(2-(2-(2-(2-(piperazin-1-yl)ethoxy)ethoxy)ethoxy)ethyl)piperazine-1 carboxylate (4e).

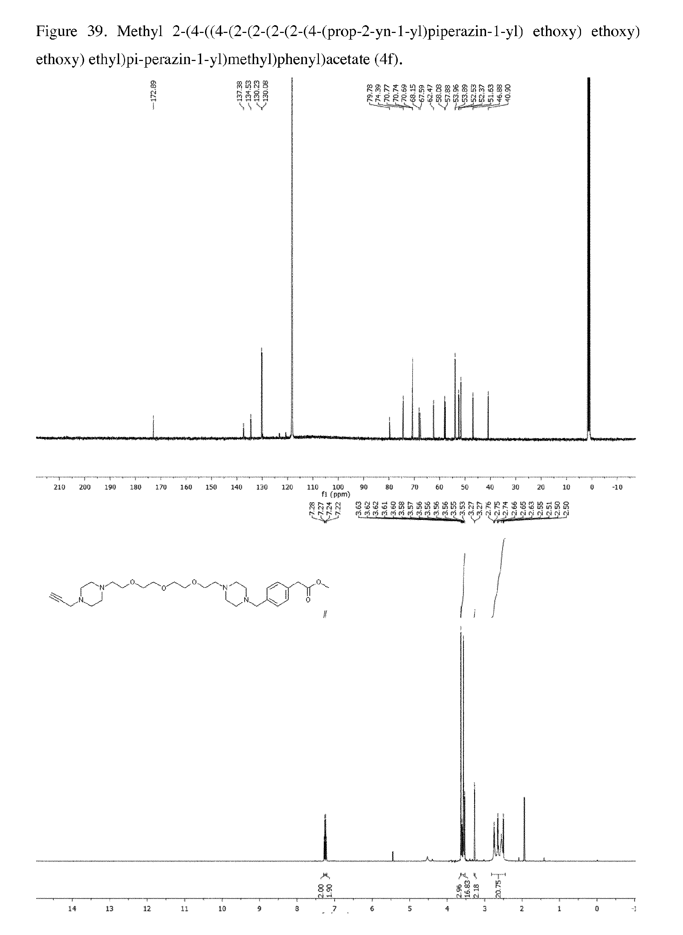

Figure 39. Shows analytical TLC, ¹H and ¹³C NMR spectra, ESI MS spectra of Methyl 2-(4-((4-(2-(2-(2-(2-(4-(prop-2-yn-1-yl)piperazin-1-yl)ethoxy)ethoxy)ethoxy)ethyl)piperazin-1-yl)methyl)phenyl)acetate (4f).

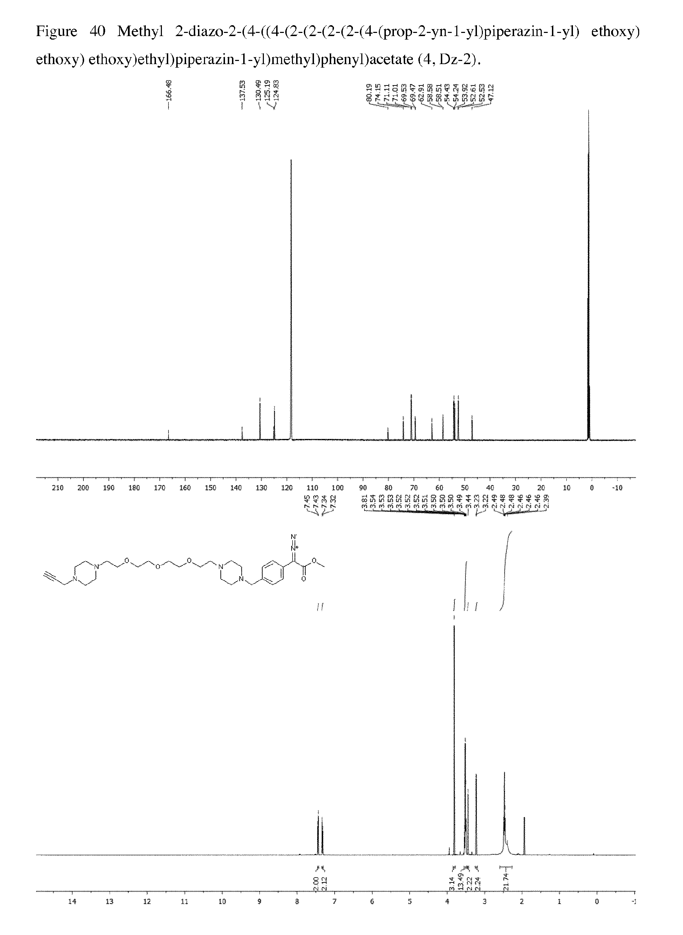

Figure 40. Shows analytical TLC, ¹H and ¹³C NMR spectra, ESI MS spectra of Methyl 2-diazo-2-(4-((4-(2-(2-(2-(2-(4-(prop-2-yn-1-yl)piperazin-1-yl)ethoxy)ethoxy)ethoxy) ethyl)piperazin-1-yl)methyl)phenyl)acetate (4, Dz-2).

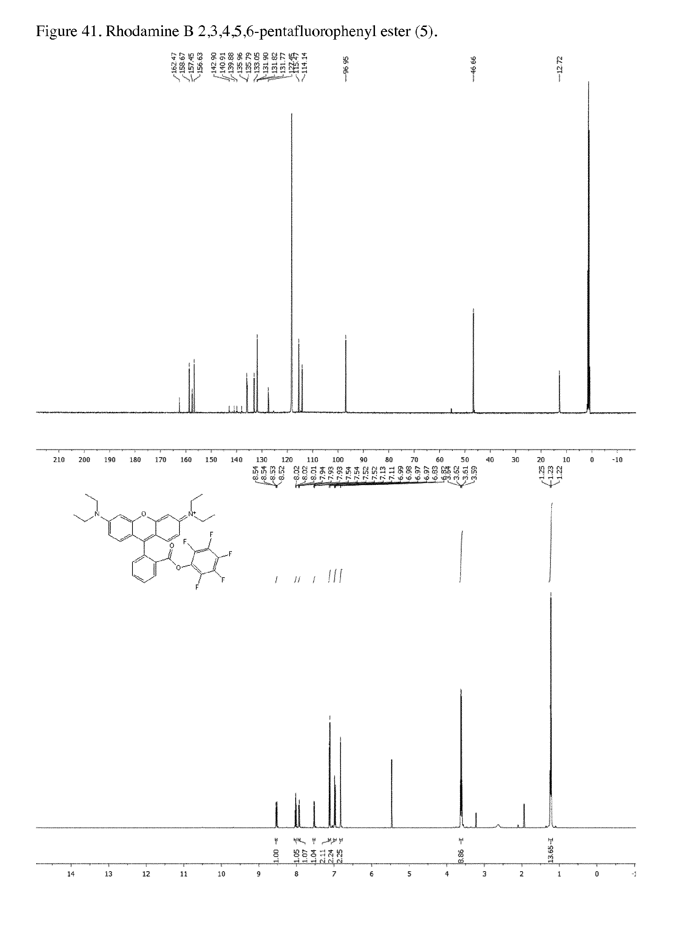

Figure 41. Shows analytical TLC, ¹H and ¹³C NMR spectra, ESI MS spectra of Rhodamine B 2,3,4,5,6-pentafluorophenyl ester (5).

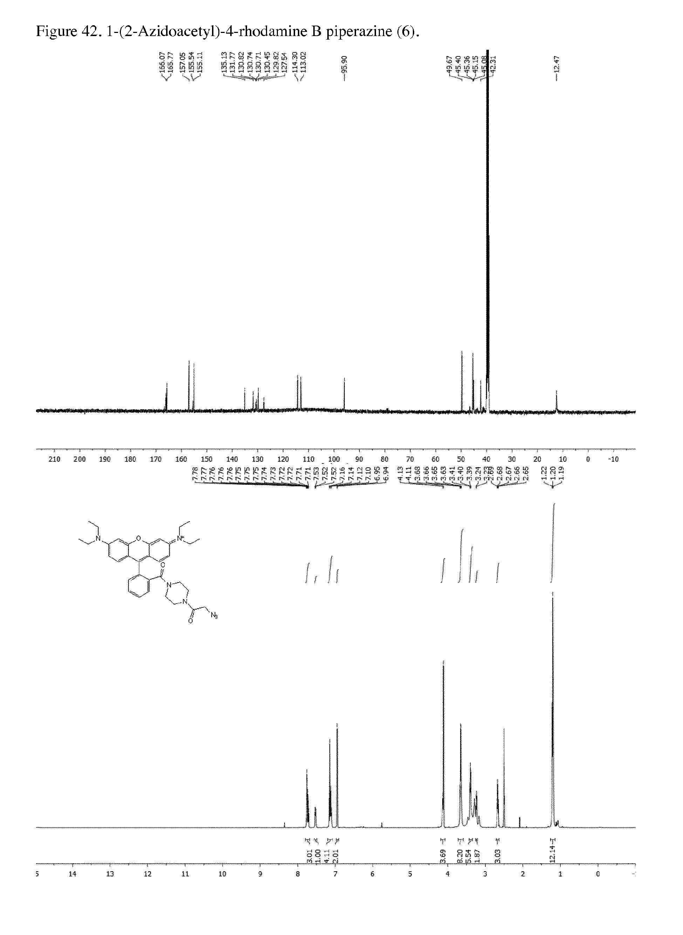

Figure 42. Shows analytical TLC, ¹H and ¹³C NMR spectra, ESI MS spectra of 1-(2-Azidoacetyl)-4-rhodamine B piperazine (6).

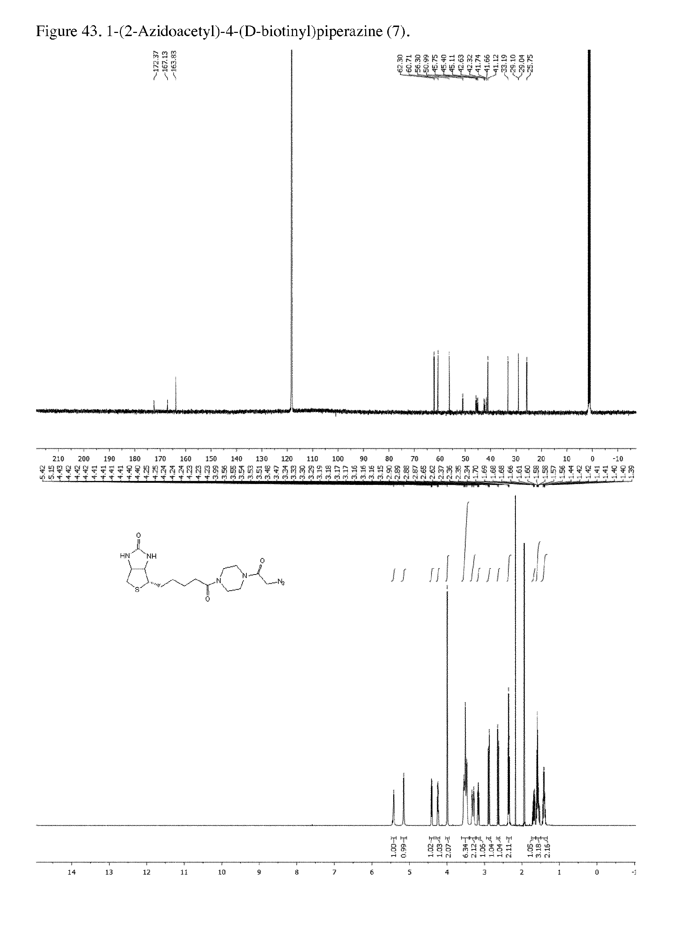

Figure 43. Shows analytical TLC, ¹H and ¹³C NMR spectra, ESI MS spectra of1-(2-Azidoacetyl)-4-(D-biotinyl)piperazine (7).

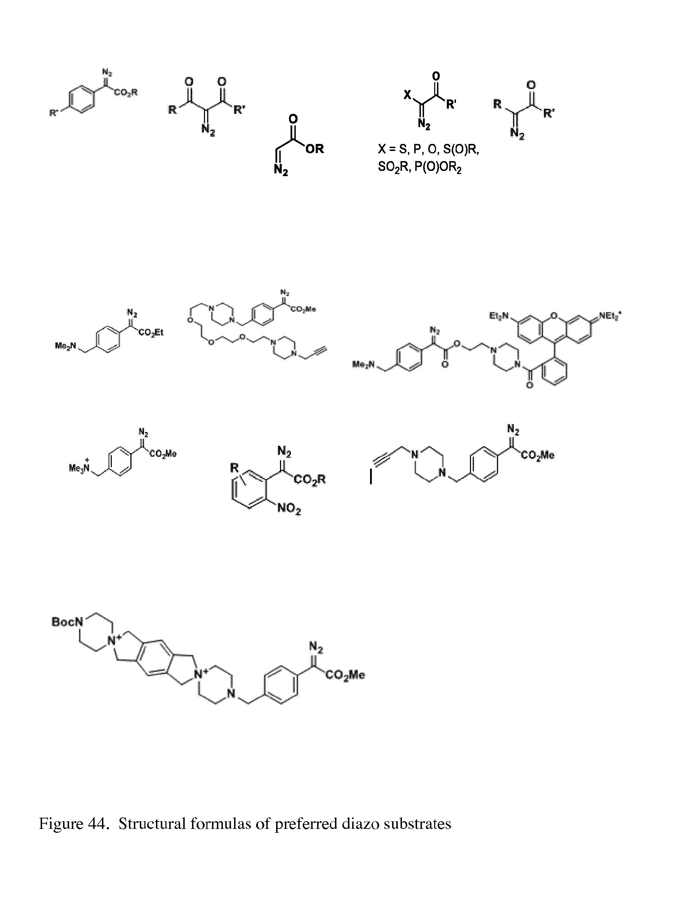

Figure 44. Shows structural formulas of preferred diazo substrates.

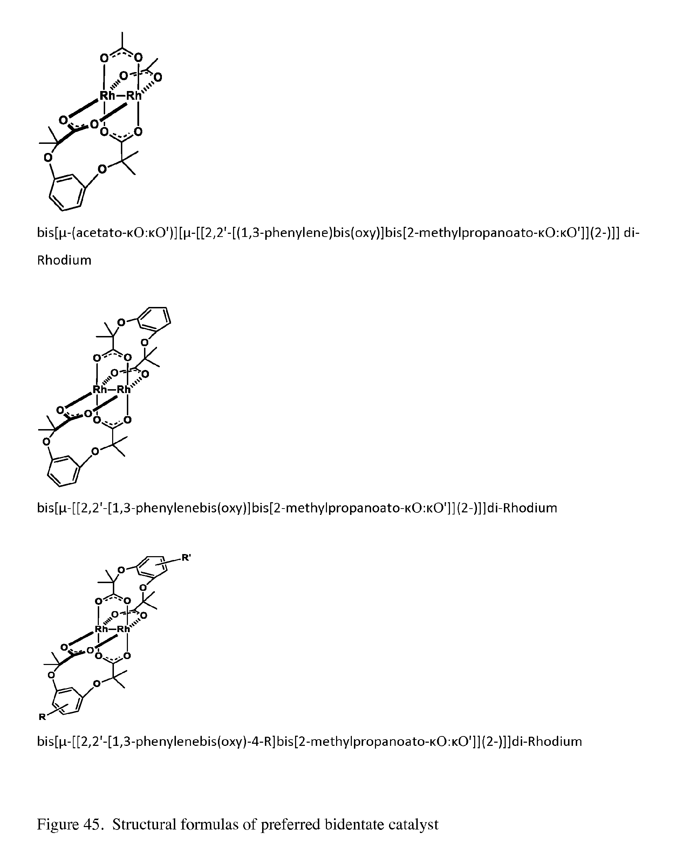

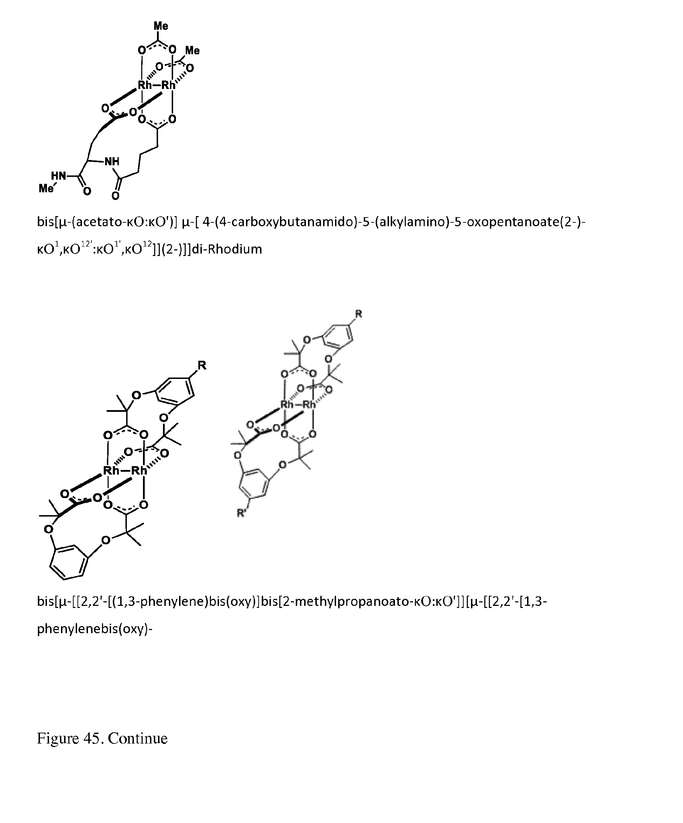

Figure 45. Shows structural formulas of preferred bidentate catalyst.

### DETAILED DESCRIPTION OF THE INVENTION

### Methods of the present invention

In one preferred aspect of the invention, a method for modifying a nucleic acid in a biological or an artificial composition is provided. This method comprises treating the composition with a rhodium catalyst and a substrate. The method according to the invention can be performed *in vivo* and *in vitro.*

The biological or the artificial composition of the present invention is treated with the catalyst and the substrate by any means standard in the art. The compositions according to the invention are preferably selected from mammalian blood, purified or partially purified blood components, blood plasma, serum, mammalian cell culture, nucleic acid sample, cell lysates, reconstituted cell components, prokaryotic cell cultures, or self-assembled nucleic acid structures.

The nucleic acid according to the present invention comprises an unpaired nucleobase, series of unpaired nucleobases, a mismatched nucleobase or series of mismatched nucleobases. Further, the nucleic acid according to the invention is preferably present within a DNA fragment, for example, genomic DNA, cDNA, and synthetic DNA fragment; within a RNA fragment, for example, whole RNA, mRNA, rRNA, siRNA, hnRNA, synthetic RNA, spliced RNA, and unspliced RNA fragment; and present in complexes with other proteins and/or with histones in chromatin. In further embodiment, the nucleic acid is Short-hairpin RNA, double-stranded RNA, or microRNAs with 3'-overhangs.

In further embodiment of the invention, a method for modifying a nucleic acid in a biological or an artificial composition is provided wherein the method comprises a rhodium catalyst and a substrate wherein the catalyst comprising a labile ligand set. A preferred labile ligand set is a bidentate ligand. A bidentate ligand preferably selected from any of the structural formulas as depicted in Figure 45 wherein R and R' can be the same or different and preferably selected, independently, from acridine, carboxlylate, alkyne, azide, ketone, aldehyde, and amide groups. An oxygen can be attached to either R or R'. The molar ratio between the said ligand and the catalyst preferably is 1 or 2.

In further embodiment of the invention, the method for modifying a nucleic acid in a biological or an artificial composition comprises a rhodium catalyst and a substrate wherein the catalyst comprising a labile ligand set is pretreated with nucleic acid leading to substitution of the ligands with substituents on the nucleic acid. This nucleic acid-catalyst construct can then be used to alkylate itself, or a complementary strand with a diazo substrate.

In the present invention, the rhodium catalysts, the rhodium catalysts comprising a labile ligands, the substrates, the diazo substrates, and their method of manufacturing are known in the art (for example, see Bonar-Law, J. Chem. Soc., Dalton Trans., 2000, 4343-4347, and J. Am. Chem. Soc., 2004, 126 (47), pp 15378-15379).

In a further preferred embodiment of the invention said substrate is a diazo substrate. A preferred diazo substrate is selected from structural formula as described in Table 4 and depicted in figure 44 wherein R and R' preferably selected from a group consisting of alkyl chains of varying length, Fluorescent molecules, Biotin tag, digoxygenin, polyethylene glycol polymers of varying length, cationic peptides such as octa-arginine and related cell-penetrating peptides, nucleic acids or nucleic acid mimics. Preferred R groups are methyl, ethyl, ethylene glycol monomethyl ether and higher polyethyleneglycol (PEG) homologues. Preferred R' groups are dialkylbenzylamines, methylketones, aromatic rings, alkylhydroxylamines, alkylhydrazines, alkylazides, and (PEG)ₙ where n = 1, 2, 3.

The substrate, preferably, the diazo substrate according to the present invention can be tailored without limitations except that the substrate, preferably, the diazo functional group is present to react with the catalyst. In one embodiment, the present invention provides a guiding motif that is incorporated into the diazo substrate. In addition, the diazo substrates as shown in tables 3 and 4, for example, which incorporate an alkyne functional group provide a handle for further modification after the nucleic acid labeling. The substrate, preferably, the diazo substrate according to the present invention can be labeled.

In another embodiment of the invention, the composition is treated with a rhodium-carbenoid. The rhodium-carbenoid preferably generated from reacting a dirhodium tetracetate with a diazo substrate.

In one embodiment of the invention, a method of directly and modifying a nucleic acid in a biological or an artificial composition is provided. The method comprises treating the composition directly with a rhodium catalyst and a substrate. Direct modification or treating the composition directly according to the invention shall mean that the catalyst contacts a native nucleic acid structure which has not been chemically treated pre- or post-synthetically to facilitate the modification. For instance, the catalyst according to this embodiment of the invention is not linked to a guiding motif. The term guiding motif generally refers to a molecular motif that is capable of binding to a nucleic acid target in a sequence-specific way.

In another embodiment of the invention, a method of selectively modifying a nucleic acid according to any of the proceeding embodiments is provided, wherein said method comprises linking a rhodium catalyst to a guiding motif, wherein the guiding motif is identical, is complementary, or hybridizes under stringent or highly stringent conditions to all or portion of the nucleic acid.

Stringent hybridization conditionsinvolve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

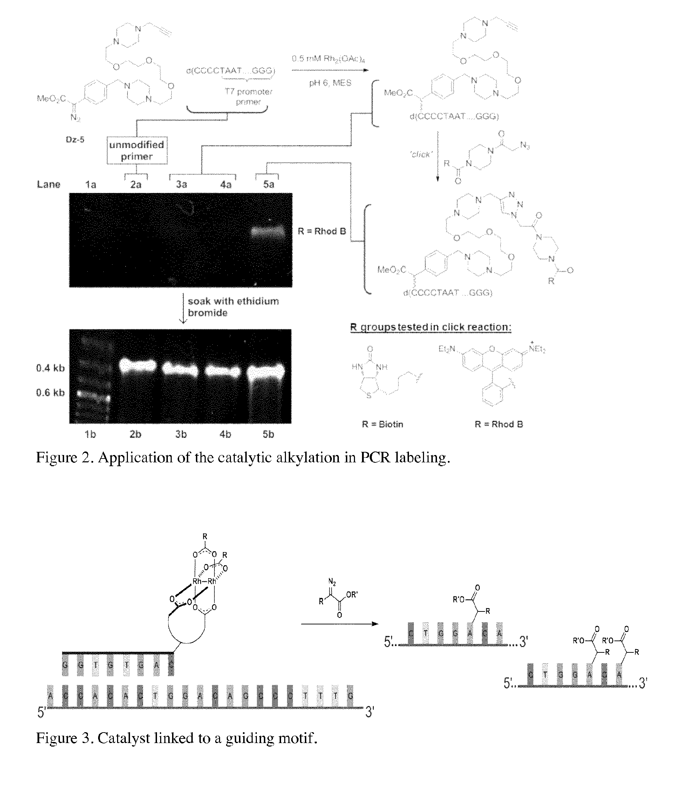

As depicted in Figure 3., for sequence specific alkylation the catalyst is linked to a guiding motif that is a nucleic acid or any other molecule capable of sequence-specific binding to nucleic acids. The guided catalyst binds to the target nucleic acid and only then the diazo molecule is activated and the target nucleic acid is modified. The rhodium catalyst can be linked to a guiding motif by any means standard in the art. It is preferred that alkylhydroxylamine/ketone coupling be used as the linking strategy. The guiding motif can be made of a nucleic acid, a locked nucleic acid, a peptide nucleic acid, or a polycationic peptide that binds to a nucleic acid according to the standard methods in art. In further embodiment of the invention, the guiding motif comprises a functional group for association with the catalyst. The functional groups for linking catalyst and guiding motif preferably are: (a) alkyl hydroxyl amine on the ligand of the catalyst and a ketone or aldehyde incorporated within the guiding motif, (b) alkylhydroxlamine on one of the ends of the guiding motif and a ketone or aldehyde on one the ligands of the catalyst, (c) a sulfhydryl on both the catalyst and the guiding motif that can be linked together via disulfide bond, (d) an azide on the guiding motif and an alkyne on one of the ligands of the catalyst, these can then be linked by copper-catalyzed azide alkyne Cycloaddition, (e) an alkyne on the guiding motif and an azide on one of the ligands of the catalyst, these can then be linked by copper-catalyzed azide alkyne Cycloaddition, and (f) a carboxylic acid on one or both ligands of the catalyst can be linked with amide coupling techniques to a free amine incorporated within the guiding motif. It is further preferred that the ligand comprises a functional group for association to the guiding motif which are selected from alkylhydroxylamine, aldehyde, carboxylic acid, alkyne, azide and more preferably ketone.

In another preferred method of the present invention the method of selectively modifying a nucleic acid further comprising the steps of: (a) embedding the catalyst in a nucleic acid sequence or a nucleic acid mimic sequence, wherein the nucleic acid or the nucleic acid mimic is a stem-loop sequence, (b) inactivating the catalyst by a deactivating ligand at the opposite end of the said stem-loop sequence, (c) contacting the inactivated and embedded catalyst with the nucleic acid in a biological or an artificial composition, wherein the nucleic acid is identical, is complementary, or hybridizes under stringent conditions to all or portion of the stem-loop sequence. The preferred guiding nucleic acid or nucleic acid mimic is peptide nucleic acid, morpholine nucleic acid, glycerol nucleic acid, and threose nucleic acid. It is preferred that said deactivating ligands for rhodium compounds are nitriles, dimethylanilines, sulfoxides, phosphines which are effective and reversible. It is further preferred that the guiding nucleic acid or nucleic acid mimic is identical, is complementary, or hybridizes under stringent conditions to all or portion of desired target nucleic acid sequence. More preferably, the guiding nucleic acid or nucleic acid mimic embodies a functional group that allows and facilitates association with the catalyst in solution. The rhodium catalyst of this embodiment preferably contains ligands with a functional group that facilitates association to said guiding motif. Preferably, this method comprises (a) a catalyst embedded in a stem-loop nucleic acid sequence or nucleic acid mimic sequence according to the previous embodiments which is held in an inactive state by a deactivating ligand at the opposite end of the said stem-loop sequence, (b) a biological or an artificial sample containing a target nucleic acid complementary to all or a portion of the stem-loop sequence binds and force open the loop creating a double-strand, thus removing the deactivating ligand from the catalyst, (c) the catalyst is then in an active state and reacts with added diazo substrate.

In another aspect of the invention, the method of selectively modifying a nucleic acid according to any embodiments of the present invention, further comprises detecting a nucleic acid in a biological or an artificial composition, comprising (a) treating the composition comprising the nucleic acid with a rhodium catalyst and a substrate, (b) contacting the treated nucleic acid in (a) with a probe specific for said treated nucleic acid to form a complex comprising said treated nucleic acid and said probe hybridized to said treated nucleic acid, and (c) detecting hybridization of said probe to said nucleic acid.

In a preferred embodiment, said detection further comprises (a) embedding the catalyst in a nucleic acid sequence or a nucleic acid mimic sequence, wherein the nucleic acid is a stem-loop sequence, (b) inactivating the catalyst by a deactivating ligand at the opposite end of the said stem-loop sequence, (c) contacting the inactivated and embedded catalyst with the nucleic acid in a biological or an artificial composition, wherein the nucleic acid is identical, is complementary, or hybridizes under stringent or highly stringent conditions to all or portion of the stem-loop sequence, (d) contacting the modified nucleic acid in (c) with a probe specific for said modified nucleic acid to form a complex comprising said modified nucleic acid and said probe hybridized to said modified nucleic acid, and (e) detecting hybridization of said probe to said nucleic acid.

Inactivating the catalyst in step (b) substantially eliminates the ability of the catalyst to react with the substrate. Contacting the inactivated and embedded catalyst with the target nucleic acid forces the deactivating ligand to be removed from the catalyst.

In further embodiment of the invention, the detection method comprises sequence-specific activation of the catalyst via a guiding motif, which then alkylates a nucleic acid according to a method of the previous embodiments. Preferably, this method comprises (a) a catalyst embedded in a stem-loop nucleic acid sequence or nucleic acid mimic sequence according to the previous embodiments which is held in an inactive state by a deactivating ligand at the opposite end of the said stem-loop sequence, (b) a biological or an artificial sample containing a target nucleic acid complementary to all or a portion of the stem-loop sequence binds and force open the loop creating a double-strand, thus removing the deactivating ligand from the catalyst, (c) the catalyst is then in an active state and reacts with added diazo compounds to generate a fluorescent signal, or to generate a molecule that can be detected enzymatically or immunohistochemically.

The detection step or method according to the above embodiments of the invention is performed by any means standard in the art. Preferably, detection is performed by fluorescence, radioactivity, coupled enzyme assay, or antibody detection (ELISA).

In another aspect of the invention, an oligonucleotide for amplifying a nucleic acid is provided. The oligonucleotide is modified according to any of the proceeding embodiments and is identical, is complementary, or hybridizes under stringent or highly stringent conditions to a nucleic acid. In another embodiment, an oligonucleotide set for amplifying a nucleic acid is provided, wherein the oligonucleotide set comprises a pair of oligonucleotides, and at least one of the pair of oligonucleotide is an oligonucleotide that is modified according to any of the proceeding embodiments.

It is preferable that the modified oligonucleotide for amplifying a nucleic acid according to the present invention is labeled. In further embodiment, the labeled modified oligonucleotide is identical, is complementary, or hybridizes under stringent or highly stringent conditions to a nucleic acid containing unpaired nucleobase, series of unpaired nucleobases, a mismatched nucleobase or series of mismatched nucleobases. In further embodiment, the modified oligonucleotide is identical, is complementary, or hybridizes under stringent or highly stringent conditions to a genetically abnormal nucleic acid.

In further embodiment, the present invention provides a method according to any of the proceeding embodiments for amplifying a nucleic acid sequence contained in a biological or an artificial composition. This method comprises (a) adding a modified oligonucleotide according to the above embodiments to the biological or an artificial composition, (b) amplifying the nucleic acid contained in the biological or an artificial composition using the modified oligonucleotide according to the above oligonucleotide embodiments.

The amplification method according to the invention is performed by any means in the art. It is preferred that the nucleic acid amplification method in (b) is performed via polymerase chain reaction (PCR), realtime PCR, quantitative PCR.

In further embodiment, said nucleic acid detection and amplification of the present invention is carried out in the presence of a mismatched and/or unpaired nucleobase and/or series of mismatched and/or unpaired nucleobases.

In another embodiment, the present invention provides a method of determining the presence or absence of a genetic abnormality in a nucleic acid contained in a biological or an artificial composition, comprising (a) amplifying the nucleic acid contained in a biological or an artificial composition by a nucleic acid amplification method according to the above embodiments, (b) measuring a fluorescence intensity before and after amplifying the target nucleic acid sequence in (a), and, (c) detecting the presence or absence of a genetic abnormality by comparing the fluorescence intensities measured in (b). In further embodiment, the present invention provides a method of determining the presence or absence of a genetic abnormality in a nucleic acid contained in a biological or an artificial composition, wherein the genetic abnormality comprises a mismatched nucleobase, series of a mismatched nucleobases, an unpaired nucleobase, a series of unpaired nucleobases, or a series of repeated nucleobases expansions.

In another embodiment, the present invention provides a nucleic acid modified according to any of the proceeding embodiments, and sequences complementary thereto. In further embodiment a nucleic acid modified according to the proceeding embodiments is Short-hairpin RNA, double-stranded RNA, or microRNAs with 3'-overhangs.

In another aspect of the invention a kit for selectively modifying a nucleic acid in a biological composition is provided. The kit comprises (a) a rhodium catalyst (b) a substrate (c) containers suitable for containing the said rhodium catalyst, a substrate, and the biological composition. In further embodiment, the kit comprises the catalyst according to the proceeding embodiments, wherein said catalyst further comprises a guiding motif, wherein the guiding motif is identical, is complementary, or hybridizes under stringent or highly stringent conditions to all or portion of the nucleic acid. In further embodiment of the kit, said guiding motif comprising a functional group for association with the catalyst. In further embodiment of the kit, the catalyst comprises a ligand that contains a functional group that facilitates association to said guiding motif. In further embodiment of the invention, a kit for nucleic acid detection and amplification according is provided wherein the kit comprises (a) a rhodium catalyst (b) a substrate (c) an oligonucleotide, or an oligonucleotide set.

The present invention provides novel uses of a rhodium catalyst and a substrate for modifying, amplifying and detecting a nucleic acid in a biological or an artificial composition.

Further, the present invention provides novel uses of a method and/or a kit according to any of the proceeding embodiments in alkylating RNA *in vivo* to alter protein production, RNA interference, and/or signaling between one or series of RNA. A nucleic acid modified according to the proceeding embodiments, wherein nucleic acid is Short-hairpin RNA, double-stranded RNA, or microRNAs with 3'-overhangs modified according to the preceding claims are used in RNA interference or antisense therapy.

In another embodiment, the present invention provides novel uses of a method, an oligonucleotide and/or a kit according to any of the proceeding embodiments in generating chemical libraries of modified RNA for use in controlling or modulating RNA interference, or diseases of RNA misregulation. The method preferably involves generating short hairpin RNAs or microRNAs or double-stranded RNAs according to established recombinant or synthetic techniques. These RNAs always have a 3'-overhang that would be freely available for reaction as described for example in Table 1, entry 5. Libraries of diazo compounds are generated through diazo transfer with a variety of commercially available ester or ketone substrates that contain an enolizable alpha-proton. Other established methods for diazo synthesis could also be used to create libraries in one step from commercial compounds. Examples include condensing a hydrazine or protected hydrazine with a ketone and then oxidizing to obtain the diazo compound.

In another embodiment, the present invention provides novel uses of a method, an oligonucleotide and/or a kit according to any of the proceeding embodiments in detecting genetic abnormality in a nucleic acid, in the diagnosis, prognosis, monitoring and/or classification of cellular proliferative disorders.

### Experiments and results:

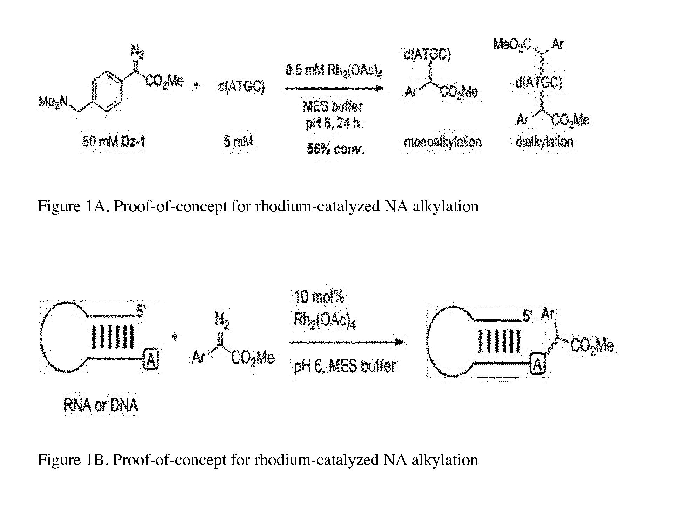

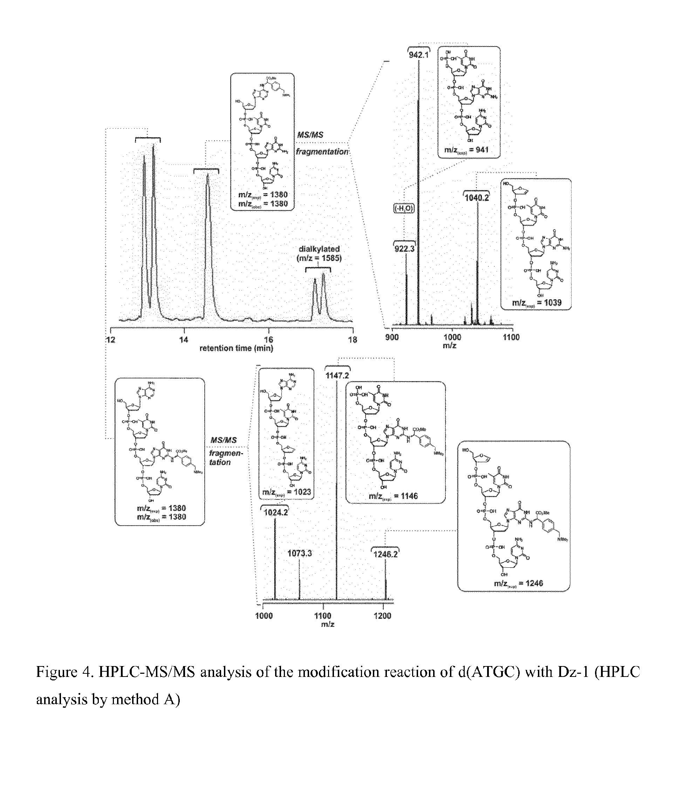

In an initial proof-of-invention experiment the simple tetradeoxynucleotide d(ATGC) was treated with 10 mol% Rh₂(OAc), and the diazo substrate Dz-1 in aqueous buffer (Figure 1A). Under these conditions the Dz-1 substrate was completely consumed after 24 h and a number of new products were observed whose masses corresponded to singly- and doubly-modified d(ATGC). Tandem MS analysis of the monoalkylation products allowed unambiguous assignment of the purine bases as the sites of modification (see, Figure 4).

With the established feasibility of targeting NAs through rhodium-carbenoid catalysis, a more comprehensive investigation of the reaction with a series of hairpin sequences was performed (Table 1). Hairpins were chosen because they contain a number of common nucleic acid (NA) structural elements in a single molecule. The first hairpin we tested contained thymidines (Ts) in its turn region and was otherwise double-stranded (entry 1, Table 1). It was established that Ts were unreactive from the experiment with d(ATGC) so this molecule allows an assessment of the susceptibility of double-stranded stretches to the alkylation. Interestingly this hairpin was completely unreactive, revealing the prospect of exploiting double strands as a type of shielding motif to enable the targeting of specific unpaired bases in a given NA. To test this possibility hairpins containing adenine as an overhang base (entries 2, 3, and 5) or in the turn-region (entry 4) were synthesized. As expected both of these motifs were viable substrates (entries 2, 3, and 5 with entry 4). The specificity of the process for unpaired NA sequences offers as described above a new strategic tool for post-synthetic NA modification and may prove practical for chemical tailoring of DNA architectures in the burgeoning field of DNA nanotechnology.

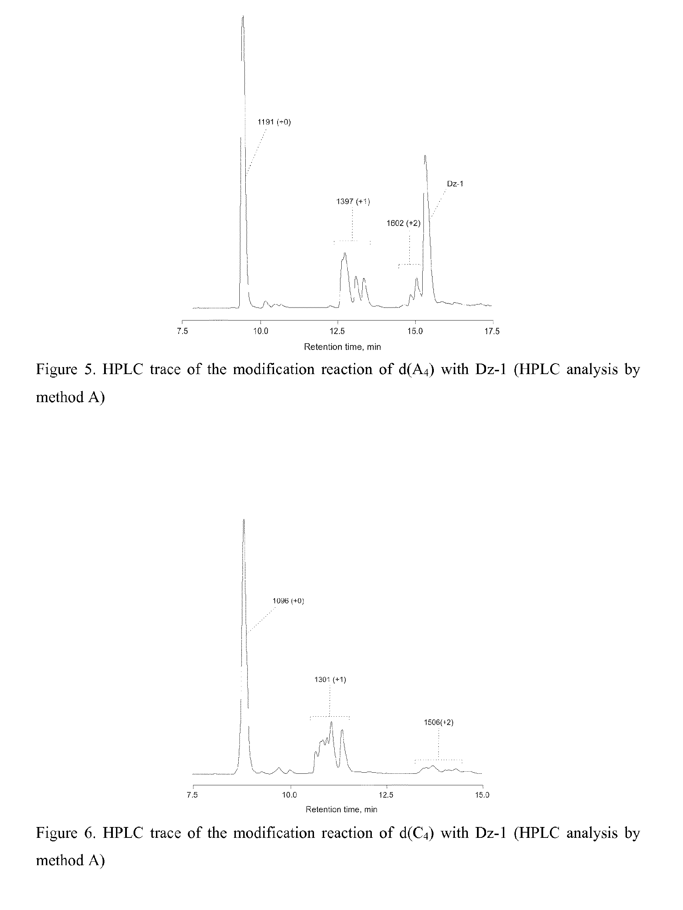

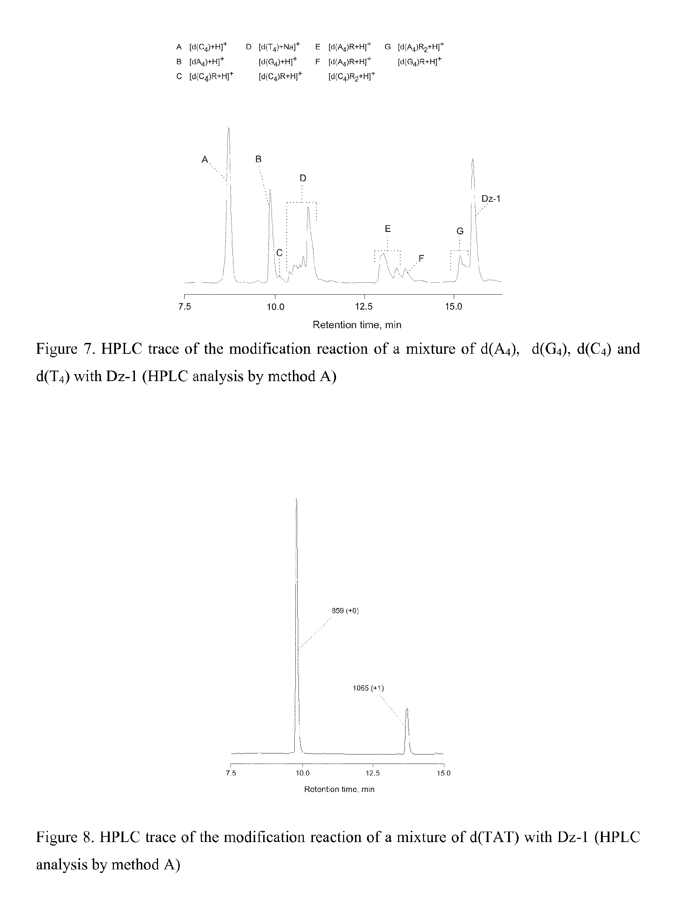

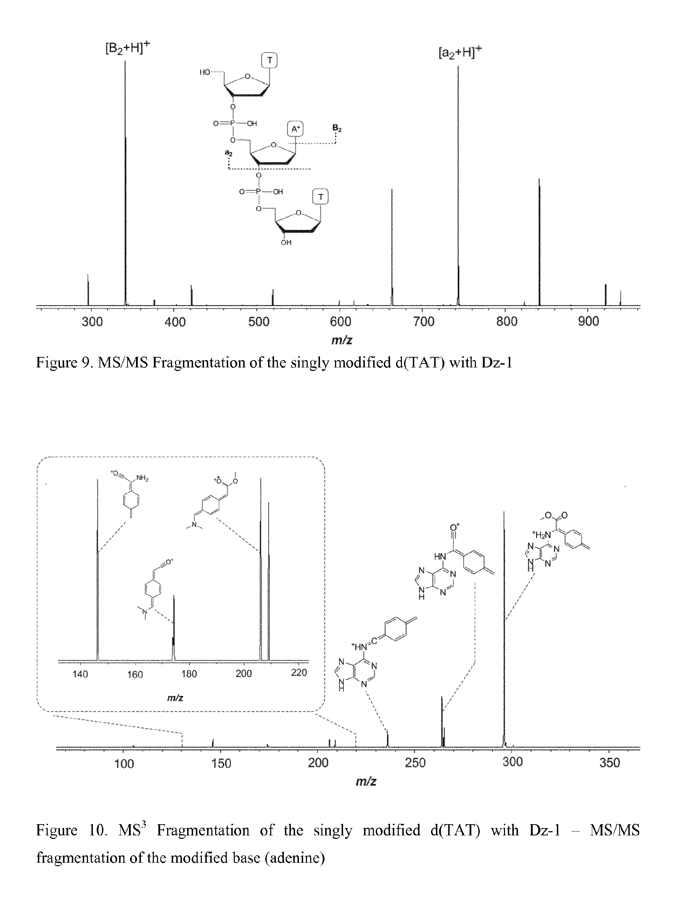

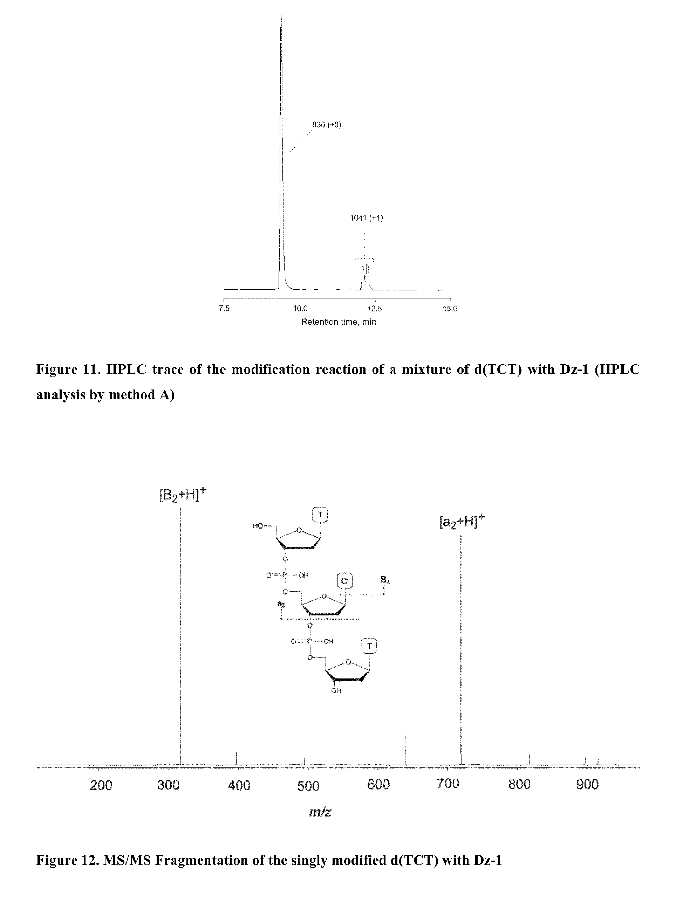

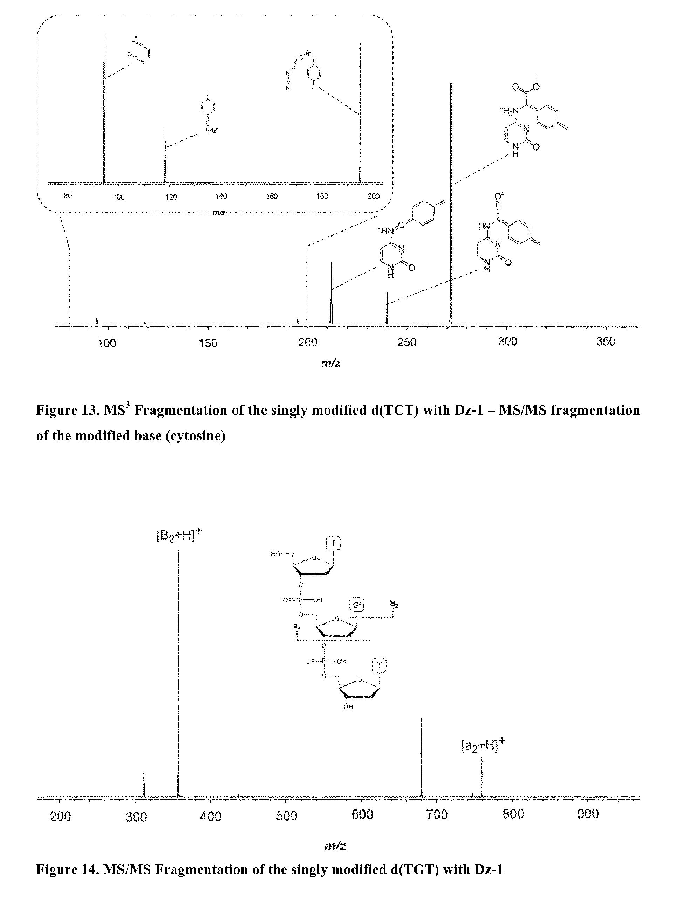

Short hairpin RNAs and short dsRNAs are molecules of high current interest because of their central role in the RNA interference (RNAi) pathway (J. Kurreck, Angew. Chem. Int. Ed. 2009, 48, 1378-1398). RNAs that participate in this pathway always contain 3'-overhangs, which are essential to trigger the assembly of the active ribonucleoprotein complex. The successful 3'-alkylation of a short hairpin RNA (entry 5, Table 1) is therefore especially significant as it establishes the method as a convergent approach to the synthesis of base-modified RNAs. Such molecules have been employed to study and modulate RNAi (H. Peacock, A. Kannan, P. A. Beal, C. J. Burrows, J. Org. Chem. 2011, 76, 7295-7300; b) A. R. Hernández, L. W. Peterson, E. T. Kool, ACS Chem. Biol. 2012), but thus far the diversity of the modified RNA pool has been limited due to the laborious linearity of solid-phase synthesis methods. The method according to the present invention alleviates many of the current problems. The preference for purine nucleobases observed for the alkylation of d(ATGC) (figure 1A) warranted a systematic evaluation of which features in the oligonucleotide primary structure were important in the modification. Homotetramers were analysed to probe the relative propensity of each base towards alkylation (entries 1-4, Table 2). Consistent with the results from the hairpin series homotetramers d(T)₄ and r(U)₄ were completely unmodified, indicating that neither the ribophosphate backbone nor T and U nucleobases are the site of reaction (The possibility that d(T)₄ is simply an inhibitor of the catalyst was excluded by a control experiment where d(T)₄ was added to the reaction of d(A)₄ and no change was observed in its alkylation profile (see, Figure 6). Additionally, MS/MS experiments confirm that trinucleotides containing C or A nested between Ts (entries 5-6, Table 2) were alkylated only at the C or A sites and never at the Ts (see, Figures 8-13). In contrast to the result with d(ATGC), where C was untouched, alkylation of d(C)₄ proceeded efficiently (entry 4, Table 2), suggesting that purine bases are alkylated preferentially but that Cs can also be targeted. The remainder of the RNAs and DNAs shown in Table 2 provide further evidence of the generality of the alkylation process.

Diazo substrate that did not lead to NA alkylation was instead predominately converted to the corresponding hydroxyl insertion product (see header in Table 2). Despite this side-reaction a significant percentage of catalyst turnovers were directed to the oligonucleotide. Considering the enormous excess of water this indicates that N-H insertion is significantly faster than hydroxyl insertion (see Selectivity Factor, Table 2). It is tenable that this preference is simply a consequence of the increased nucleophilicity of the amine towards the intermediate rhodium carbenoid, but two observations speak to a more intimate role of the NA: First, while the alkylation of d(TGT) was slow and often led to the formation of a precipitate, a mixture of d(TAT) and d(TGT) led to smooth alkylation of both NAs. Second, while Dz-1 only slowly delivered hydroxyl insertion under the standard reaction conditions (<10% conv. in 24 h), when d(TAT) was present not only is the NA alkylated but the rate of the hydroxyl insertion side-reaction also increases significantly. The first step in the catalytic cycle for rhodium carbene formation is generally accepted to be the interaction of the negatively polarized carbon of the diazo molecule with the empty d_{z}² orbital in the axial position of the rhodium complex (M. C. Pirrung, H. Liu, A. T. Morehead, J. Am. Chem. Soc. 2002, 124, 1014-1023). With this in mind it seems unlikely that a direct nitrogen coordination from the ON substrate to the catalyst is responsible for the selectivity observed as this would likely inhibit carbene formation (M. C. Pirrung, H. Liu, A. T. Morehead, J. Am. Chem. Soc. 2002, 124, 1014-1023*).* These results clearly demonstrate that certain NAs can affect the catalytic reaction and this augurs well for the potential of controlling the site of alkylation through the incorporation of particular NA sequences.

Given the unreactivity of Ts and Us, it was determined that the site of alkylation with the remaining nucleobases was the exocyclic N-H (C. J. Moody, Angew. Chem. Int. Ed. 2007, 46, 9148-9150). Alkylation of N³ of adenine and N⁷ of guanine were deemed unlikely based on analogy to the classic experiments of Maxam and Gilbert (A. M. Maxam, W. Gilbert, Proc. Natl. Acad. Sci. USA 1977, 74, 560-564). They established that alkylations at these positions-and not the exocyclic nitrogens-render the resulting modified bases highly susceptible to depurination: it was observed no such change in stability (Alkylated nucleotides were monitored at pH 5 and pH 8.5 for 24 h and their HPLC profiles were unchanged). More direct evidence for N-H insertion is garnered from MS³ experiments for the three trinucleotides d(TAT), d(TGT), and d(TCT). The ions observed after the fragmentation of these alkylated trinucleotides are in full agreement with those expected for exocyclic N-H insertion products, and are difficult to reconcile with any other potential reaction pathway (see, Figures 8-13) (S. S. Jensen, X. Ariza, P. Nielsen, J. Vilarrasa, F. Kirpekar, J. Mass Spectrom. 2007, 42, 49-57).

As the results collected in Table 3 indicate the process showed little dependence on the structure of the diazo substrate or reaction conditions. In terms of substrate scope maintaining water solubility at the concentrations 25-50 mM typically needed to achieve efficient alkylation. Efficient alkylation according to the invention means >20% conversion. If the nucleic acid concentration is low (<mM) then an increase in diazo concentration or multiple additions of diazo can still deliver efficient conversion. Although the substrate could be customized for any specific application, the propargyl-containing molecules Dz-4 and Dz-5 (entries 8-9, Table 3) should prove particularly versatile because they allow the Cu-catalyzed azide-alkyne cycloaddition to be employed to generate diverse structures from a single diazo precursor-a feature demonstrated by the 'click' reaction of both a rhodamine fluorophore and a biotin derivative (see bottom-right of Figure 2).

The practical potential of the process was highlighted through the generation of fluorescently labeled PCR amplicons. A modified T7 promoter primer (Figure 2) was first alkylated with Dz-5 and then used in a PCR reaction. We expected a PCR to be successful despite the unspecific alkylation profile with single-stranded NAs since double-helix formation and polymerase extension should only occur with those primers that get alkylated near their 5'-end. Indeed this seems to be the case as robust PCR amplification is seen in all samples that employ alkylated T7 promoter primers (Fig. 2, Lanes 3-5). The experiment in lane 5 is particularly important as it highlights the potential of the technique for generating labeled genes; here the propargyl group is linked through a 'click' reaction to a rhodamine derivative prior to the PCR. The amplification product of this plasmid PCR is clearly visible with a standard UV lamp before the addition of ethidium bromide, indicating that labeled amplicons are accessible through this simple technique. Figure 2 depicts the following: top: The T7 promoter primer, which contains a short 5' extension, is alkylated under standard conditions with the propargyl-containing diazo substrate Dz-5. Gel: The gel is run without ethidium bromide to allow visualization of fluorescently labeled products (Lanes 1a-5a) and then soaked with ethidium bromide to visualize all NA species (Lanes 1b-5b). Lane 1: 100 bp DNA ladder. Lane 2: Control reaction with unmodified T7 promoter primer and plasmid DNA. Lane 3: T7 promoter primer is alkylated with Dz-5 and this entire crude mixture is then employed in a PCR reaction with plasmid DNA. Lane 4: Same as lane 3 except the alkylated T7 promoter primer is purified before carrying out the PCR. Lane 5: The alkylated primer is subjected to a click reaction with the rhodamine B azide substrate and then employed in the PCR. Bottom-right: Both biotin- and rhodamine B-azide derivatives are viable substrates for the click reaction.

The methods according to the invention as disclosed above establish the feasibility of employing rhodium catalysis to achieve the direct alkylation of native NA structures. Moreover its predictable selectivity profile allows the strategic targeting of unpaired nucleobases such as those present in single strands, bulge regions, and overhangs. One of the key features of the present methods of the invention are their simplicity and directness: the catalyst is commercially available, the diazo compounds are readily prepared, and native NAs are viable substrates. The preference for N-H insertion observed here, as well as by others in the protein labeling field (J. M. Antos, J. M. McFarland, A. T. Iavarone, M. B. Francis, J. Am. Chem. Soc. 2009,131, 6301-6308) is intriguing. Particularly considering the myriad of other functional groups available and the enormous excess of water present in these reactions. The new catalytic alkylation methods according to the disclosed embodiments of the invention offer a powerful strategy in the labeling and modification of NA derivatives which can be applied toward tailoring of DNA architectures and in the labeling of NAs for biological study.

### General Procedures

All reagents and solvents used were of analytical grade. Buffers were prepared with ultrapure water. All chemicals were purchased from Sigma-Aldrich, Fluka or Acros and used as received. Analytical TLC was performed on Silica gel 60 F₂₅₄ pre-coated aluminium sheets. Flash chromatography was performed on Silica gel 60 40-63 mm (230-400 mesh) (SiliCycle, Quebec). Solid-phase oligonucleotide synthesis was carried out on an Expedite 8909 nucleic acid synthesis system (PerCeptive Biosystems). ¹H and ¹³C NMR spectra were acquired on a Bruker AvanceIII+ (500 MHz) spectrometer at 298 K. Chemical shifts relative to TMS were referenced to the solvent's residual peak and are reported in ppm. Proton-decoupled ¹⁹F spectra were acquired on a Bruker AvanceIII+ (400 MHz) at 298 K. EI MS were obtained on a Finnigan MAT 95 device. ESI MS spectra were obtained on a Bruker Esquire3000plus spectrometer by direct injection in positive polarity of the ion trap detector. MALDI TOF analyses were carried out on a Bruker Microflex mass-spectrometer in linear negative mode using 3 hydroxypicolinic acid/ammonium citrate, pH 6.0 or 2,4,6 trihydroxyacetophenone/ ammonium citrate, pH 6.0 matrices. All HPLC procedures were carried out on an Agilent 1100 LC system equipped with a Chromolith Performance RP18e 4.6 x 100 mm column (Merck) using 100 mM triethylammonium acetate (pH 7.1)/acetonitrile gradients as a mobile phase. Elution was carried out at a flow rate of 1 ml/min using one of the following methods. Method A: 0-16 % acetonitrile in 12 min, 16-80 % acetonitrile in 3 min, 80 % acetonitrile in 2 min, or method B: 0-16 % acetonitrile in 12 min, 16-80 % acetonitrile in 15 min, 80 % acetonitrile in 2 min. Detection was carried out by monitoring the absorbance of the column effluent at 254 nm. Nucleic acids were obtained by any means standard in the art.

### Chemical syntheses

### Diazo substrate synthesis strategy

Both key α-diazo carbonyl compounds **Dz-1** and **Dz-2** were synthesized from a common precursor, methyl 4-(2-bromomethyl)phenylacetate. In the case of **Dz-1** substitution of the bromine with dimethylamine and introduction of the α-diazo carbonyl function via nucleophilic diazo transfer afforded the target compound (Figure 33A). The preparation of **Dz-2** included the introduction of an acetylene function for the subsequent 'click' chemistry reactions and a tetra(ethylene glycol) motif to improve the water solubility of the target diazo substrate ( Figure 33B).

**Methyl 2-(4-(bromomethyl)phenyl)acetate (2).** 4-(Bromomethyl)phenylacetic acid (0.2 g, 0.87 mmol) was mixed with dry methanol (2 ml) and dry benzene (2 ml) under nitrogen. Trimethylsilyldiazomethane (2 mol/l in hexanes) was added drop-wise at continuous stirring until stable yellow color of the solution was obtained (the reaction vessel should be vented as gas evolution takes place). The mixture was stirred for 15 min at room temperature, then evaporated and dried under high vacuum to yield the pure compound as a colorless oil (0.21 g, 0.86 mmol, quant.). TLC (n-hexane:Et₂O 4:1 v/v) R_{f} =0.36. ¹H NMR (500.1 MHz, CDCl₃) d, ppm: 7.36 (d, J = 8.1 Hz, 2H), 7.26 (d, J = 8.2 Hz, 2H), 4.48 (s, 2H), 3.69 (s, 3H), 3.62 (s, 2H). ¹³C NMR (125.8 MHz, CDCl₃) d, ppm: 171.73, 136.68, 134.28, 129.75, 129.31, 52.16, 40.86, 33.21. EI MS: 104.1 [M-Br-COOMe]⁺, 163.1 [M-Br]⁺, 183.0, 185.0 [M-COOMe]⁺, 242.0, 244.0 [M]⁺ (see Figure 33).

**Methyl 2-diazo-2-(4-((dimethylamino)methyl)phenyl)acetate (3, Dz-1).** Compound 2 (0.2 g, 0.82 mmol) was dissolved in 2 ml of absolute ethanol, dimethylamine in absolute ethanol (2 ml, 5.6 mol/l) was added and the mixture was stirred at room temperature for 30 min. The volatiles were then removed under vacuum and the residue was partitioned between ethyl acetate and saturated sodium bicarbonate solution. The organic phase was washed with brine, dried with anhydrous sodium sulfate and evaporated under vacuum. The remaining yellow oil was dried under high vacuum, and then mixed with p-acetamidobenzenesulfonyl azide (pABSA, 0.216 g, 0.90 mmol) in 5 ml of dry acetonitrile under nitrogen. DBU (0.187 g, 1.23 mmol) was added to the solution with continuous stirring and the mixture was stirred for 24 h at room temperature. The mixture was then evaporated under vacuum and the residue purified by flash chromatography on Si60 in DCM/methanol to afford a yellow-orange oil (0.082 g, 0.35 mmol, 43 %). TLC (DCM:methanol 10:1 v/v) R_{f}-0.33. ¹H NMR (500.1 MHz, CDCl₃) d, ppm: 7.43 (d, J = 8.5 Hz, 2H), 7.33 (d, J = 8.5 Hz, 2H), 3.86 (s, 3H), 3.41 (s, 2H), 2.23 (s, 6H). ¹³C NMR (125.8 MHz, CDCl₃) d, ppm: 165.76, 136.63, 129.74, 124.07, 123.94, 63.87, 52.01, 45.33. ESI MS: Calcd for C₁₂H₁₆N₃O₂⁺ [M+H]⁺ 234.1, found 234.1(see Figure 34).

**tert-Butyl4-(2-(2-(2-(2-hydroxyethoxy)ethoxy)ethoxy)ethyl)piperazine-1-carboxylate (4b).** 1-Boc-piperazine (0.362 g, 1.9 mmol) was dissolved in 15 ml of dry acetonitrile under nitrogen. K₂CO₃ (1.0 g, 7.0 mmol) was added, the mixture was heated up to reflux and compound **4a** (prepared as previously described¹) (0.5 g, 1.9 mmol) as a solution in 5 ml of dry acetonitrile was introduced with continuous stirring. The mixture was refluxed for 18 h, then filtered and the filtrate evaporated under vacuum. The residue was purified by flash chromatography on Si60 in DCM/methanol to afford the target product as a colorless oil (0.551 g, 1.5 mmol, 80 %). TLC (DCM:methanol 20:1 v/v) R_{f}=0.14. ¹H NMR (500.1 MHz, CDCl₃) d, ppm: 3.68 (ddd, J = 4.5 Hz, J = 3.5 Hz, J = 2.0 Hz, 2H), 3.55-3.64 (m, 12H), 3.39-3.41 (m, 4H), 2.55 (dd, J = 5.7 Hz, J = 5.7 Hz, 2H), 2.40-2.42 (m, 4H), 1.41 (s, 9H). ¹³C NMR (125.8 MHz, CDCl₃) d, ppm: 154.69, 79.55, 72.70, 70.58, 70.24, 70.22, 68.64, 61.59, 57.85, 53.32, 43.96, 42.93, 28.40. ESI MS: Calcd for C₁₇H₃₄N_{z}NaO₆⁺ [M+Na]⁺ 385.2, found 385.3(see Figure 35).

**tert-Butyl4-(2-(2-(2-(2-(tosyloxy)ethoxy)ethoxy)ethoxy)ethyl)piperazine-1-**carboxylate (4c). Compound 4b (0.550 g, 1.5 mmol) was mixed with triethylamine (0.307 g, 3.0 mmol) and 4-dimethylaminopyridine (0.019 g, 0.16 mmol) in 30 ml of dry acetonitrile under nitrogen. p-Toluenesulfonyl chloride (0.318 g, 1.7 mmol) as a solution in 10 ml of dry acetonitrile was introduced at continuous stirring. The mixture was stirred at room temperature for 48 h, then evaporated under vacuum and residue was purified by flash chromatography on Si60 in DCM/methanol to yield the target product as a colorless oil (0.517, 1.0 mmol, 66 %). TLC (DCM:methanol 20:1 v/v) R_{f} =0.28. ¹H NMR (500.1 MHz, CDCl₃) d, ppm: 7.78 (d, J = 8.3 Hz, 2H), 7.33 (d, J = 8.5 Hz, 2H), 4.14 (ddd, J = 4.5 Hz, J = 4.0 Hz, J = 3.0 Hz, 2H), 3.67 (ddd, J = 5.0 Hz, J = 4.0 Hz, J = 3.5 Hz, 2H), 3.57-3.61 (m, 10H), 3.40-3.42 (m, 4H), 2.58 (dd, J = 6.0 Hz, J = 6.0 Hz, 2H), 2.41-2.43 (m, 7H), 1.44 (s, 9H). ¹³C NMR (125.8 MHz, CDCl₃) d, ppm: 154.75, 144.81, 132.99, 129.84, 127.99, 79.58, 70.77, 70.63, 70.52, 70.37, 69.23, 68.86, 68.70, 57.81, 53.37, 44.02, 43.00, 28.43, 21.67. ESI MS: Calcd for C₂₄H₄₁N₂O₈S⁺ [M+H]⁺ 517.3, found 517.2(see Figure 36).

**tert-butyl4-(2-(2-(2-(2-bromoethoxy)ethoxy)ethoxy)ethyl)piperazine-1-carboxylate (4d).** LiBr (0.695 g, 8.0 mmol) was mixed with dry acetone (12 ml) and heated up to reflux under nitrogen. Compound **4c** (0.517 g, 1.0 mmol) as a solution in 3 ml of dry acetone was added with continuous stirring and the mixture was refluxed until the reaction was complete as judged by TLC (2 h). The solution was then cooled to room temperature and evaporated under vacuum. The residue was mixed with 30 ml of DCM and the white precipitate that formed was removed by filtration. The filtrate was taken up to dryness and the residue was purified by flash chromatography on Si60 in DCM/methanol to afford the target product as a colorless oil (0.419 g, 1.0 mmol, 98 %). TLC (DCM:methanol 20:1 v/v) R_{f} =0.28. ¹H NMR (500.1 MHz, CDCl₃) d, ppm: 3.81 (dd, J = 6.3 Hz, J = 6.3 Hz, 2H), 3.61-3.69 (m, 10H), 3.47 (dd, J = 6.3 Hz, J = 6.3 Hz, 2H), 3.42-3.44 (m, 4H), 2.60 (dd, J = 5.9 Hz, J = 5.9 Hz, 2H), 2.43-2.45 (m, 4H), 1.45 (s, 9H). ¹³C NMR (125.8 MHz, CDCl₃) d, ppm: 154.77, 79.59, 71.23, 70.70, 70.62, 70.58, 70.42, 68.91, 57.84, 53.38, 40.25, 30.31, 28.44. ESI MS: 425.2, 427.1 [M+H]⁺, 447.1, 449.1 [M+Na]⁺. ESI MS: Calcd for C₁₇H₃₄BrN₂O₅⁺ [M+H]⁺ 425.2, 427.2, found 425.2, 427.1; Calcd for C₁₇H₃₃BrN₂NaO₅⁺ [M+Na]⁺ 447.2, 449.2, found 447.1, 449.1(see Figure 37).

**tert-Butyl4-(2-(2-(2-(2-(piperazin-1-yl)ethoxy)ethoxy)ethoxy)ethyl)piperazine-1-carboxylate (4e).** Compound **4d** (0.400 g, 0.93 mmol) was dissolved in 5 ml of dry acetonitrile and added to a stirring mixture of piperazine (0.404 g, 4.7 mmol) and K₂CO₃ (0.650 g, 4.7 mmol) in 15 ml of dry acetonitrile. The mixture was refluxed for 18 h under nitrogen. After cooling to room temperature the solids were filtered off, the filtrate was evaporated under vacuum and the residue was purified by flash chromatography on Si60 in DCM/methanol containing 0.5 % triethylamine. The final product was evaporated two times from toluene and dried under high vacuum to give a colorless oil (0.285 g, 0.66 mmol, 71 %). TLC (DCM:methanol 5:1 v/v containing 0.5 % Et₃N) R_{f} =0.22. ¹H NMR (500.1 MHz, CDCl₃) d, ppm: 3.56-3.62 (m, 12H), 3.39-3.41 (m, 4H), 2.87 (dd, J = 5.0 Hz, J = 5.0 Hz, 4H), 2.55 (ddd, J = 11.0 Hz, J = 5.5 Hz, J = 5.0 Hz, 4H), 2.38-2.46 (m, 9H), 1.42 (s, 9H). ¹³C NMR (125.8 MHz, CDCl₃) d, ppm: 154.72, 79.55, 70.58, 70.39, 70.38, 68.69, 68.82, 58.33, 57.81, 54.71, 53.36, 45.88, 44.00, 43.10. ESI MS: Calcd for C₂₁H₄₃N₄O₅⁺ [M+H]⁺ 431.3, found 431.3; Calcd for C₂₁H₄₂N₄NaO₅⁺ [M+Na]⁺ 453.3, found 453.2; Calcd for C₂₁H₄₂KN₄O₅⁺ [M+K]⁺ 469.3, found 469.2(see Figure 38).

**Methyl2-(4-((4-(2-(2-(2-(2-(4-(prop-2-yn-1-yl)piperazin-1-yl)ethoxy)ethoxy) ethoxy)ethyl)pi-perazin-1-yl)methyl)phenyl)acetate (4f).** tert-Butyl 4-(2-(2-(2-(2-(piperazin-1-yl)ethoxy)-ethoxy)ethoxy)ethyl)piperazine-1-carboxylate **4e** (0.10 g, 0.23 mmol) was mixed with K₂CO₃ (0.20 g) in dry acetonitrile (5 ml) under nitrogen. Methyl 4-(bromomethyl)phenylacetate **2** (0.056 g, 0.23 mmol) as a solution in 5 ml of acetonitrile was added with continuous stirring. The mixture was stirred at room temperature until the reaction was complete as judged by TLC (2 h). The solids were then filtered off, the filtrate was evaporated to dryness under vacuum and the remaining colorless oil was dissolved in 5 ml of dry DCM. 2,6-Lutidine (0.093 g, 0.87 mmol) was added and the mixture was cooled in an ice-water bath under nitrogen. TMSOTf (0.111 g, 0.5 mmol) was introduced drop-wise with continuous stirring. The ice bath was removed and the mixture was stirred at ambient temperature until complete as judged by TLC (1 h). The solvent was removed under vacuum and the remaining oil was dissolved in dry acetonitrile (5 ml), K₂CO₃ (0.26 g) was added and the mixture was stirred for 10 min under nitrogen. Propargyl bromide (0.056 g, 0.36 mmol) as a solution in toluene was then added and the mixture was stirred at room temperature until complete as judged by TLC (1 h). The solvent was taken up to dryness under vacuum and the residue was purified by flash chromatography on Si60 in DCM/methanol to afford the desired product as a colorless oil (0.098 g, 0.18 mmol, 80 %). TLC (DCM:methanol 10:1 v/v) R_{f}=0.10. ¹H NMR (500.1 MHz, CD₃CN) d, ppm: 7.27 (d, J = 8.2 Hz, 2H), 7.23 (d, J = 8.2 Hz, 2H), 4.53 (br s, 1H), 3.63 (s, 3H), 3.53-3.62 (m, 16H), 3.27 (d, J = 2.5 Hz, 2H), 2.50-2.76 (m, 21 H). ¹³C NMR (125.8 MHz, CD₃CN) d, ppm: 172.89, 137.38, 134.53, 130.23, 130.08, 79.79, 74.39, 70.78, 70.74, 70.69, 68.15, 67.59, 62.47, 58.08, 57.89, 53.96, 53.89, 52.53, 52.38, 51.63, 46.88, 40.90. ESI MS: Calcd for C₂₉H₄₇N₄O₅⁺ [M+H]⁺ 531.4, found 531.3; Calcd for C₂₉H₄₆N₄NaO₅⁺ [M+Na]⁺ 553.3, found 553.3; Calcd for C₂₉H₄₆KN₄O₅⁺ [M+K]⁺ 569.3, found 569.3(see Figure 39).

**Methyl2-diazo-2-(4-((4-(2-(2-(2-(2-(4-(prop-2-yn-1-yl)piperazin-1-yl)ethoxy) ethoxy) ethoxy) ethyl)piperazin-1-yl)methyl)phenyl)acetate (4, Dz-2).** Compound **4f** (0.090 g, 0.17 mmol) was mixed with p-acetamidobenzensulfonyl azide (0.062 g, 0.26 mmol) in 5 ml of dry acetonitrile under nitrogen. DBU (0.103 g, 0.68 mmol) was introduced drop-wise with continuous stirring. The mixture was stirred for 48 h at room temperature, then evaporated to dryness under vacuum. Purification of the residue by flash chromatography on Si60 in DCM/methanol yielded the target product as an orange-yellow oil (0.028 g, 0.05 mmol, 30 %). TLC (DCM:methanol 10:1 v/v) R_{f}=0.22. ¹H NMR (500.1 MHz, CD₃CN) d, ppm: 7.44 (d, J = 8.5 Hz, 2H), 7.33 (d, J = 8.5 Hz, 2H), 3.81 (s, 3H), 3.49-3.54 (m, 12H), 3.44 (s, 2H), 3.22 (d, J = 2.5 Hz, 2H), 2.39-2.49 (m, 21H). ¹³C NMR (125.8 MHz, CD₃CN) d, ppm: 166.48, 137.53, 130.49, 125.19, 124.83, 80.19, 74.15, 71.11, 71.01, 69.53, 69.47, 62.90, 58.57, 58.51, 54.42, 54.24, 53.92, 52.61, 52.52, 47.12. ESI MS: Calcd for C₂₉H₄₅N₆O₅⁺ [M+H]⁺ 557.3, found 557.4; Calcd for C₂₉H₄₄N₆NaO₅⁺ [M+Na]⁺ 579.3, found 579.3; Calcd for C₂₉H₄₄KN₆O₅⁺ [M+K]⁺ 595.3, found 595.3(see Figure 40).

**Rhodamine B 2,3,4,5,6-pentafluorophenyl ester (5).** Rhodamine B (0.600 g, 1.35 mmol), 2,3,4,5,6-pentafluorophenol (0.250 g, 1.36 mmol) and 4-(N,N-dimethylamino)pyridine (0.021 g, 0.17 mmol) were dissolved in 20 ml of dry DCM under nitrogen and N,N'-dicyclohexylcarbodiimide (0.327 g, 1.59 mmol) as a solution in 2 ml of DCM was added with continuous stirring. The mixture was stirred at room temperature until complete as judged by TLC (1.5 h). It was then evaporated and the residue purified by column chromatography on Si60 in DCM/methanol to afford the target product as a deep-purple solid (0.610 g, 1.0 mmol, 74 %). TLC (DCM:methanol 10:1 v/v) R_{f}=0.20. ¹H NMR (500.1 MHz, CD₃CN) d, ppm: 8.53 (dd, J = 8.0 Hz, J = 1.0 Hz, 1H), 8.02 (ddd, J = 7.6 Hz, J = 7.6 Hz, J = 1.3 Hz, 1H), 7.93 (ddd, J = 7.8 Hz, J = 7.8 Hz, J = 1.3 Hz, 1H), 7.53 (dd, J = 7.6 Hz, J = 1.0 Hz, 1H), 7.12 (d, J = 9.5 Hz, 2H), 6.98 (dd, J = 9.6 Hz, J = 2.5 Hz, 2H), 6.82 (d, J = 2.5 Hz, 2H), 3.62 (q, J = 7.2 Hz, 8H), 1.23 (t, J = 7.1 Hz, 12H). ¹³C NMR (125.8 MHz, CD₃CN) d, ppm: 162.47, 158.67, 157.44, 156.63, 142.91 (m, CF), 140.91 (m, CF), 139.88 (m, CF), 137.93 (m, CF), 135.96, 135.79, 133.05, 131.90, 131.82, 131.77, 127.45, 115.47, 114.14, 96.95, 46.66, 12.72. ¹⁹F NMR (376.5 MHz, CD₃CN) d, ppm: -154.76 (d, J = 16.8 Hz), -159.47 (t, J = 21.0 Hz), -164.16 (dd, J = 16.9 Hz, J = 21.9 Hz). ESI MS: Calcd for C₃₄H₃₀F₅N₂O₃⁺ [M]⁺ 609.2, found 609.3(see Figure 41).

**1-(2-Azidoacetyl)-4-rhodamine B piperazine (6).** Compound **5** (0.035 g, 0.06 mmol) was dissolved in 5 ml of dry acetonitrile and N-(2-azidoacetyl)piperazine² (0.014 g, 0.08 mmol) as a solution in 1 ml of acetonitrile was added with continuous stirring under nitrogen. The mixture was stirred for 24 h at room temperature, then evaporated under vacuum and the residue purified by column chromatography on Si60 in DCM/methanol to afford a deep purplish-violet waxy solid (0.016 g, 0.03 mmol, 48 %). TLC (DCM:methanol 5:1 v/v) R*_{f}*=0.32. ¹H NMR (500.1 MHz, DMSO-*d₆*) d, ppm: 7.71-7.78 (m, 3H), 7.52-7.53 (m, 1H), 7.10-7.16 (m, 4H), 6.95 (d, J = 2.3 Hz), 4.11-4.13 (m, 3H), 3.66 (q, J = 7.2 Hz, 8H), 3.39-3.41 (m, 3H), 3.22-3.24 (m, 1H), 2.65-2.69 (m, 3H), 1.20 (t, J = 7.2 Hz, 12H). ¹³C NMR (125.8 MHz, DMSO-*d₆*) d, ppm: 166.07, 165.77, 157.05, 155.54, 155.11, 135.13, 131.77, 130.82, 130.74, 130.71, 130.45, 129.82, 127.54, 114.30, 113.02, 95.90, 49.67, 45.40, 45.36, 45.15, 45.08, 42.31, 12.47. ESI MS: Calcd for C₃₄H₄₀N₇O₃⁺ [M]⁺ 594.3, found 594.4(see Figure 42).

**1-(2-Azidoacetyl)-4-(D-biotinyl)piperazine (7).** D-Biotin 4-nitrophenyl ester (0.068 g, 0.19 mmol) was suspended in 5 ml of dry acetonitrile and N-(2-azidoacetyl)piperazine² (0.042 g, 0.25 mmol) as a solution in 1 ml of acetonitrile was added with continuous stirring under nitrogen. The mixture was stirred for 24 h at room temperature, then evaporated under vacuum and the residue purified by column chromatography on Si60 in DCM/methanol to afford a colorless oil which was crystallized from chloroform (0.062 g, 0.16 mmol, 84 %). TLC (DCM:methanol 10:1 v/v) R*_{f}*=0.23. ¹H NMR (500.1 MHz, CD₃CN) d, ppm: 5.42 (br s, 1H), 5.15 (br s, 1H), 4.41 (dddd, J = 6.5 Hz, J = 5.0 Hz, J = 2.5 Hz, J = 1.0 Hz, 1H), 4.24 (ddd, J = 8.0 Hz, J = 4.5 Hz, J = 2.0 Hz, 1H), 3.99 (s, 2H), 3.47-3.56 (m, 6H), 3.28-3.34 (m, 2H), 3.17 (ddd, J = 7.9 Hz, J = 6.9 Hz, J = 4.5 Hz, 1H), 2.88 (dd, J = 12.7 Hz, J = 5.0 Hz, 1H), 2.63 (d, J = 12.7 Hz, 1H), 2.35 (dd, J = 8.1 Hz, J = 7.0 Hz, 2H), 1.65-1.72 (m, 1H), 1.53-1.63 (m, 3H), 1.38-1.44 (m, 2H). ¹³C NMR (125.8 MHz, CD₃CN) d, ppm: 172.37, 167.13, 163.83, 62.30, 60.71, 56.30, 50.99, 45.75, 45.40, 45.11, 42.63, 42.32, 41.74, 41.66, 41.12, 33.19, 29.10, 29.04, 25.75. ESI MS: 418.2 [M+Na]⁺. ESI MS: Calcd for C₁₆H₂₅N₇NaO₃S⁺ [M+Na]⁺ 418.2, found 418.2(see Figure 43).

### Oligonucleotide syntheses and purification

Solid-phase oligonucleotide synthesis was carried out on 1-µmol CPG columns using standard phosphoramidite chemistry with 0.3 M 5-benzylthio-1-H-tetrazole as activator. The DNA oligonucleotides were cleaved from the support with 32 % (v/v) aqueous ammonia for 2 h at room temperature and deprotected for 18 h at 55 °C, then freeze-dried and purified by micropreparative HPLC using method A. The RNA oligonucleotides were synthesized using TBDMS protection strategy for the 2'-OH group. Cleavage from the support was done in 32 % aqueous ammonia/ethanol 3:1 (v/v) for 2 h at room temperature followed by deprotection for 12 h at 55 °C. The samples were freeze-dried and the TBDMS groups were cleaved with triethylamine/triethylamine trihydrofluoride/N-methylpyrrolidine 1.5:2:3 (v/v/v) for 2 h at 65 °C. The crude oligoribonucleotides were isolated by precipitation with 3 M NaOAc (1/10 volume) and n-butanol (4 volumes) for 2 h at -78 °C followed by centrifugation (15,000 x g, 10 min). The pellets were washed with 70 % (v/v) ethanol and dried under high vacuum. The samples were further purified by micropreparative HPLC using method A. The identity of all synthesized oligonucleotides was confirmed by ESI or MALDI TOF MS.

### Rh(II)-Catalyzed oligonucleotide modification using a-diazocarbonyl compounds

A number of a-diazocarbonyl substrates were screened as potential candidates for the target reaction of oligonucleotide modification (Table 4). Compounds **1-6** all proved to be efficient in modifying various oligonucleotide substrates. Water solubility, however, posed an issue as only entries **1 (Dz-1), 2** and **5 (Dz-2)** were sufficiently water-soluble under the conditions of the analysis. Increasing the bulkiness of the ester moiety appeared to have a negative effect on the yields of modified oligonucleotide as for entry **2** the efficacy of modification was reduced twice in comparison to the methyl ester counterpart (entry **1)** and with the compound from entry **7** only trace amounts of modified oligonucleotide were isolated.

**General procedure for Rh₂(OAc)₄-catalyzed oligonucleotide modification using a-diazocarbonyl compounds.** Typically 10 or 20 ml reaction mixtures containing 5 mM oligonucleotide, 500 mM Rh₂(OAc)₄ and 50 mM a-diazocarbonyl compound in 100 mM MES buffer, pH 6.0 were kept at 20 °C for 24-72 h. Analysis of the reaction products was carried out by micropreparative HPLC-separation of 5-ml aliquots of the reaction mixtures using method A. The collected peak fractions were further assessed by ESI-MS or MALDI TOF.

**Modification of DNA hairpin with a 5'-adenine overhang.** The hairpin-forming oligonucleotide d(ACG GAA TTC CGT TTT TCG GAA TTC CG) containing a restriction site for EcoRI and a 5'-adenine overhang was alkylated using diazo compound **Dz-1** according to the general procedure. The reaction mixture (10 ml) was separated by micropreparative HPLC using method A and the isolated peak fractions were analyzed by MALDI TOF and freeze-dried. The two singly-modified fractions obtained, M1 and M2, were digested with EcoRI (Roche, 30 units per mg oligonucleotide) in 1×SuRE/Cut Buffer H for restriction enzymes (Roche) for 4 h at 20°C. ESI MS and MS/MS of the obtained fragments confirmed that the alkylation takes place at the 5'-adenine overhang in both cases.

**Oligonucleotide labeling by Rh₂(OAc)₄-catalyzed acetylene-tagging followed by Cu(I)-catalyzed click chemistry.** The oligonucleotide d(TTT ATT TGT TTC TTT) was alkylated using diazo compound **Dz-2** according to the general procedure. The reaction mixture (20 ml) was separated by micropreparative HPLC using method A and the isolated peak fractions were freeze-dried. The main fraction of modified oligonucleotide was further tagged with the Rhodamine B azide or Biotin-azide (see Fig. 2) via Cu(I)-catalyzed click chemistry according to the general protocol of Hong et al. (Hong, V., Presolski, S. I., Ma, C. & Finn, M. G. Analysis and Optimization of Copper-Catalyzed Azide-Alkyne Cycloaddition for Bioconjugation. Angewandte Chemie International Edition 48, 9879-9883, doi:10.1002/anie.200905087 (2009). The reaction mixtures contained 250 mM acetylene-tagged oligonucleotide, 10 mM azide, 500 mM THPTA ligand, 100 mM CuSO₄ and 10 mM sodium ascorbate in 100 mM potassium phosphate buffer, pH 7.0. The reactions were carried out at 20 °C. The reaction progress was checked by analytical HPLC using method B. Upon completion of the reaction (typically 1.5-2 h) 10-ml aliquots were separated by micropreparative HPLC and the components of the peak fractions were identified by MALDI TOF.

**Fluorescent labeling of PCR primers by Rh₂(OAc)₄-catalyzed acetylene-tagging followed by Cu(I)-catalyzed click chemistry.** The 5'-extended T7 promoter primer d(CCC CTA ATA CGA CTC ACT ATA GGG) was alkylated using **Dz-2** according to the general procedure. The reaction mixture (20 ml) was separated by micropreparative HPLC using method A and the isolated peak fractions were freeze-dried. The total fraction of modified oligonucleotide was further tagged with compound 12 as described previously for oligonucleotide d(TTT ATT TGT TTC TTT). Upon completion of the reaction (2 h) 10-ml aliquots were separated by micropreparative HPLC and the total fraction of fluorescently-labeled primer was freeze-dried.

**PCR test experiment.** The PCR amplification of the 213-488 region of the pET-19b vector with a 170-bp sequence cloned between the NdeI and XhoI sites was tested with unmodified, acetylene-tagged, and fluorescently-labeled primer. The final concentration of the unmodified and the acetylene-tagged primers in the PCR mixtures was 2 mM, and 10 mM for the fluorescently-labeled one. The amplification products were analyzed by gel electrophoresis in 2% agarose in TAE buffer without ethidium bromide. The gels were visualized by transillumination with UV-light. In the absence of ethidium bromide only the band corresponding to the fluorescently labeled primer was visible. The gels were then soaked in TAE buffer containing ethidium bromide to visualize all nucleic acid containing species.

### Example of methods of manufacturing the catalyst comprising a labile ligand.

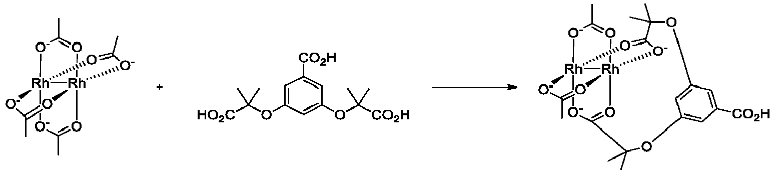

### Procedure based on: Bonar-Law, J. Chem. Soc., Dalton Trans., 2000, 4343-4347 Scale: 0.113 mmol of Rh₂(OAc)₄

Rh₂(OAc)₄ and the triacid were combined in a 25 mL round bottomed flask equipped with a stir bar. N,N-Dimethylaniline (5 mL) was added, a vigreux was attached and the reaction mixture was heated to 145 °C for 3 h, whereupon the mixture turned black. After 3.5 h a TLC (DCM/EtOAc 20:1) indicated 3 new spots. The reaction mixture was poured into DCM/HCl 2M (15 mL each) and the organic phase was separated. The organic layer was extracted with 2M HCl (2 x 15 mL) and the combined organics were then washed with water (2 x 20 mL). The combined organics were dried over MgSO₄, filtered and concentrated to give 20 mg of a green semisolid. Since the amount was too low, the still greenish water phase was extracted with DCM/MeCN (4:1, 40 mL) whereupon the color of the organic phase changed to purple (Rh-MeCN interaction). The organics were dried over MgSO₄, filtered and concentrated to give 131 mg of a green semisolid. Both portions showed the same 3 spots on the TLC so they were combined to give 151 mg of a crude green semisolid. Purified by flash chromatography: dry loaded on Silica in DCM. 15/1 cyclohexane/EtOAc, then 7/1, then 1/1, then pure EtOAc. Some green residue stayed on top of silica column. Back isolation by washing it out from the top with EtOAc. Silica was then washed with EtOAc and filtered. Washed with pure MeCN (purple), later MeOH. Solvent evaporated to give 32 mg of a crude purple solid, 55 mg of a green solid obtained after MeOH wash of the silica. Both analyzed by ESI (product inside with dimethylaniline as Ligand). Therefore all combined and MeCN added, extracted with 2 M HCl (see Reference), organic phase separated, dried over Na₂SO₄ and concentrated to give 38 mg of a dark green solid consistent with desired product according to NMR and Mass-spectroscopy.

Second ligand substitution:

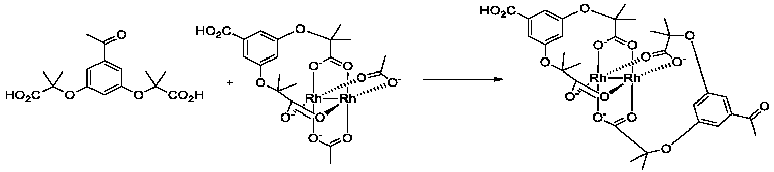

### Procedure from: J. Am. Chem. Soc., 2004, 126 (47), pp 15378-15379, Supporting Scale: 0.059 mmol of mono-ligated complex

The non purified Rh2-complex from first ligand substitution (35 mg) was transferred into a 4 mL microwave vial equipped with a stirring bar and 1.5 mL of 1,2-dichloroethane were added (sonication). To this solution was added a portion of diacid (7 mg) and the vial was closed with the suited cap and heated up in an oil bath (130°C). After 35 min the vial was removed from the oil bath and cooled down to rt (5 min, waterbath). Another portion of diacid (7 mg) was added and the vial was sealed and heating at 130°C was resumed. After another 35 min the vial was again removed from the oil bath, cooled down and another portion of diacid (5 mg) was added. The vial was sealed again and heating at 130°C was resumed for 35 min. After cooling down the vial to rt in a water bath, a TLC (1:1 Cyhex/EtOAc) indicated new spots and remaining keto-diacid. The reaction was cooled to rt (let it stand) whereupon a green solid setteled. The supernatant was separated and evaporated to give 30 mg of a green solid. ESI-MS (neg mode) indicated only non reacted keto-diacid, Rh₂(OAc)₄+N,N-Dimethylaniline and Starting Material. The remaining suspension was evaporated to dryness and resulted in 26 mg of a dark green solid. ESI-MS (neg mode) indicated the desired product with some other masses. TLC analysis (4:1 cyhex/EtOAc) of the solid indicated 3 different products. The crude (26 mg) was dissolved in MeOH (dry load) and subjected to a 5 g silica prepacked column equilibrated in (4:1 cyhex/EtOAc). The mixture was eluted with 100 mL (4:1 cyhex/EtOAc), 50 mL (2:1), 50 mL (1:1), 50 mL (1:2) and 50 mL pure EtOAc. The remaining colored material was eluted with pure MeOH.

### F7-10 turquoise solid, 4 mg, dirhodium with two keto-diacid ligands

### F15-23 green solid, 12 mg, desired product

### Experiment for sequence specific alkylation:

Synthesizing a short nucleic acid complementary to a 9-base pair region of the target sequence and then coupling the last base at the 5'-end has a 5'-amine, a-hydroxylamine acetic acid to the amine before cleaving from the solid support. Mixing a target nucleic acid sequence with the guiding strand in a 5:1 ratio to allow duplex formation. Adding the rhodium complex containing a ketone on one of the aromatic rings of the bidentate ligand followed by the diazo substrate. The catalyst self-assembles through oxime formation between catalyst ketone and the 5'-hydroxylamine of the guiding strand. Adding the diazo which alkylates the target strand after forming a carbene with the catalyst. The catalyst dissociates from the target strand and binds with a different molecule of the target strand for further alkylation.

### Experiment for nucleic acid triggered catalyst activation and detection:

Synthesizing a nucleic acid incorporating a stem-loop structure with an a-hydroxylamine acetic acid at the 5'-end and a dimethylaniline at the 3'-end. Adding the rhodium complex containing a ketone on one of the aromatic rings of the bidentate ligand and a complex forms through oxime formation with the 5'-terminal hydroxylamine. The dimethylaniline coordinates to the vacant axial orbital of the rhodium complex thereby deactivating the catalyst. Adding the Diazo substrate but no reaction occurs due to the deactivating effect of the tethered ligand. Adding a nucleic acid which is complementary to the loop region of the stem-loop sequence. The stem-loop is therefore unfolded to allow the double-helix to form between the loop and the added nucleic acid, consequently the deactivating ligand is dissociated from the catalyst and the catalyst can now react with the diazo substrate to generate a fluorescent product. The diazo substrate is a substituted phenol derivative which undergoes cyclization to a fluorescent coumarin product upon rhodium carbene formation. The appearance of the fluorescent signal therefore confirms the presence of the target nucleic acid.

### OTHER EMBODIMENTS

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery.

Such modifications are also intended to fall within the scope of the appended claims.

**Table 1. Structure-specific alkylation of hairpin DNA and RNA**

| | | | | | |
|---|---|---|---|---|---|
| 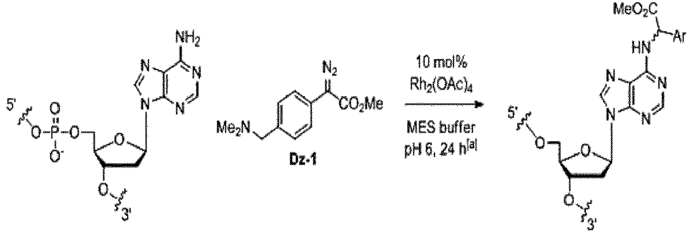 | | | | | |

| Entry^{[a]} | Sequence | Structure | Conv [%]^{[b]} | Product | Significance |
|---|---|---|---|---|---|
| 1 | d(CGAACGTTT TTCGTTCG) | 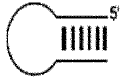 | 0 | no reaction | double-stranded sequences are unreactive |
| 2 | d(CGAACGTTT TTCGTTCGA) | 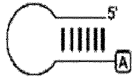 | 21 | 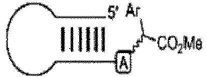 | 3' overhang regions can be targeted |
| 3^{[c]} | d(ACGGAAT TCCGTTTTT CGGAATTCCG) | 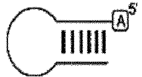 | 19 | 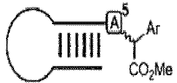 | 5° overhang regions can be targeted |
| 4 | d(CGAACGTTA TTCGTTCG) | 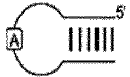 | 5 | 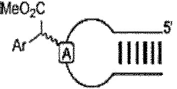 | turn regions/bulges can be targeted |
| 5 | r(CUAGCAUUU UUUGCUAGA) | 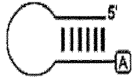 | 19 | 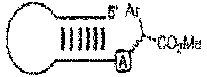 | short hairpin RNAs can be targeted |

| | | | | | |
|---|---|---|---|---|---|
| [a] Conditions: [NA] = 5 mM, **[Dz-1]** = 50 mM, [Rh₂(OAc)_{4]} = 0.5 mM, [MES] = 100 mM. [b] Conversions can be improved by adding a second bolus of **Dz-1,** shown are the results of a single addition. [c] Site of alkylation confirmed by tandem MS analysis of the fragments resulting from a restriction digest. | | | | | |

**Table 2. Scope of rhodium catalyzed alkylation with single-stranded DNAs and RNAs**

| | | | | | | |
|---|---|---|---|---|---|---|
| 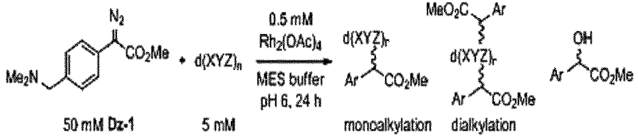 | | | | | | |

| Entry | Substrate | Total Alkylation Products | | Conv. [%]^{[a]} | Conv. **Dz-1** [%]^{[a]} | Selectivity Factor (N-H/O-H)^{[b]} |
|---|---|---|---|---|---|---|
| | | monoalkylation | dialkylation | | | |
| 1 | d(T)₄ | 0 | 0 | 0 | 20 | ∼0 |
| 2 | r(U)₄ | 0 | 0 | 0 | 30 | ∼0 |
| 3 | d(A)₄ | 4 | 2 | 39 | 40 | — |
| 4^{[c]} | d(C)₄ | 6 | 6 | 45 | >98 | — |
| 5^{[d]} | D(TAT) | 2 | 0 | 20 | >98 | 282 |
| 6^{[c][d]} | d(TCT) | 2 | 0 | 16 | >98 | 210 |
| 7^{[d]} | 4(ATGC) | 4 | 2 | 56 | >98 | — |
| 8 | r(ACUGCU) | 3 | 0 | 68 | >98 | 9970 |
| 9 | d(CTCTCT) | 5 | 1 | 56 | >98 | — |
| 10 | d(CTC TCT) | 2 | 0 | 32 | >98 | 1550 |
| 11 | 4(CTG GCT) | 6 | 0 | 42 | >98 | 1050 |
| 12 | D(TTT ATT TGT TTC TTT) | 4 | 2 | 37 | >98 | — |

| | | | | | | |
|---|---|---|---|---|---|---|
| [a] Conversion determined by HPLC. [b] Approximated from the ratio of products multiplied by the effective concentration of N-H vs. O-H insertion sites. Only calculated for reactions that do not give dialkylation. [c] **Dz-1** is completely consumed after 3 h. [d] Site of alkylation determined by MS/MS analysis. | | | | | | |

**Table 3. Scope of diazo substrates and reaction conditions in nucleic acid alkylation**

| | | | | | |
|---|---|---|---|---|---|
| 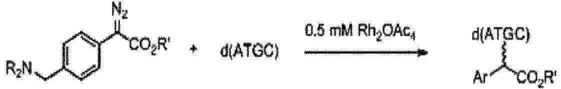 | | | | | |

| Entry | | Diazo substrate | [Diazo] (mM)^{[s]} | Buffer | Conv [%]^{[b]} |
|---|---|---|---|---|---|
| 1 | Dz-1 | 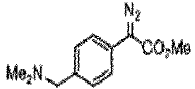 | 50 | pH 6, 100 mM MES | 56 |
| 2 | Dz-1 | | 50 | pH 6, 75 MM t-BuNHOH^{[c]} | 52 |
| 3 | Dz-1 | 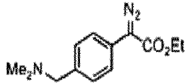 | 50 | pH 6, 100 mM KP, | 52 |
| 4 | Dz-2 | | 50 | pH 6, 100 mM MES | 24 |
| 5 | Dz-3 | 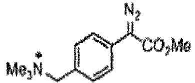 | 50 | pH 6, 100 mM MES | 48 |
| 6 | Dz-3 | | 50 | PH 6.5, 100 mM KP, | 51 |
| 7 | Dz-3 | | 50 | pH 7 5. 100 mM KP, | 50 |
| 8 | Dz-4 | 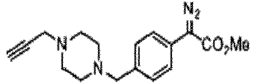 | 25 | pH 6, 100 mM MES | 30 |
| 9^{[d]} | Dz-5 | 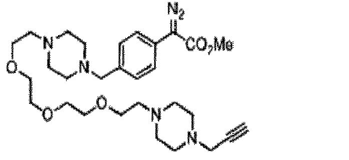 | 50 | pH 6, 100 mM MES | 51 |
| 10 | Dz-6 | 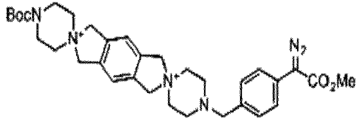 | 25 | pH 6, 100 mM MES | 40 |
| 11 | Dz-7 | 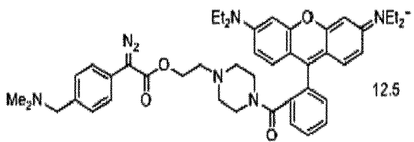 | | pH 6, 100 mM MES | <5 |

| | | | | | |
|---|---|---|---|---|---|
| [a] Concentrations below 50 mM indicate substrates of low solubility. [b] Conversion determined by HPLC analysis. [c] *t*-BuNHOH has previously been shown to impact aqueous rhodium catalyzed reactions.^{[9]} [d] d(TTT ATT TGT TTC TTT) was used instead of d(ATGC). MES = 2-(N-morpholino)ethanesulfonic acid. KP*ₜ* = potassium phosphate buffer. | | | | | |

**Table 4. α-Diazocarbonyl substrates - potential substrates for the oligonucleotide modification reaction**

| **Entry** | **Diazo substrate** | **Effective concentration in the reaction mixture^{a}, mM** | **Oligonucleotide substrate** | **Yield of alkylated oligonucleotide, %** |
|---|---|---|---|---|
| 1 | 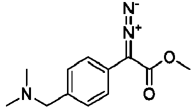 | 50 | d(ATG C) | 56 |
| 2 | 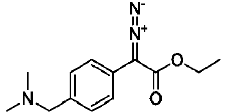 | 50 | d(ATG C) | 24 |
| 3 | 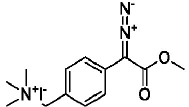 | 50 | d(ATG C) | 48 |
| 4 | 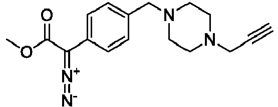 | 25^{b} | d(ATG C) | 30 |
| 5 | 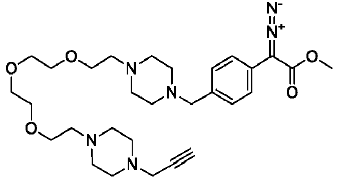 | 50 | d(TTT ATT TGT TTC TTT) | 51 |
| 6 | 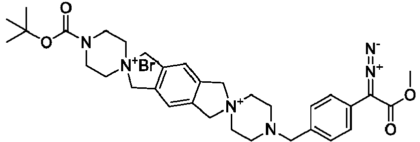 | 25 | d(ATG C) | 40 |
| 7 | 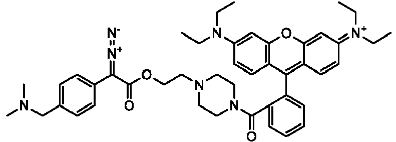 | 12.5^{b} | d(ATG C) | < 5 |

| | | | | |
|---|---|---|---|---|
| ^{a} **Reaction conditions** 5 mM oligonucleotide, 10 mol% Rh₂(OAc)₄ with respect to the oligonucleotide, 100 mM MES, pH 6.0, 24 h at room temperature. The reaction mixture contained 50 % (v/v) ethylene glycol as a co-solvent. | | | | |

## Claims

1. A method of selectively modifying a nucleic acid in a biological or an artificial composition, said method comprising the step of treating the composition with a rhodium catalyst and a substrate.

2. A method of selectively modifying a nucleic acid according to claim 1, wherein said catalyst further comprising a labile ligand set.

3. A method of selectively modifying a nucleic acid according to any of the proceeding claims, wherein said method comprising linking a rhodium catalyst to a guiding motif, wherein the guiding motif is identical, is complementary, or hybridizes under stringent or highly stringent conditions to all or portion of the nucleic acid.

4. A method of selectively modifying a nucleic acid according to any of the proceeding claims, further comprising the steps of:
a. embedding the catalyst in a nucleic acid sequence or a nucleic acid mimic sequence, wherein the nucleic acid or the nucleic acid mimic is a stem-loop sequence;
b. inactivating the catalyst by a deactivating ligand at the opposite end of the said stem-loop sequence; and
c. contacting the inactivated and embedded catalyst with the nucleic acid in the biological or an artificial composition, wherein the nucleic acid is identical, is complementary, or hybridizes under stringent conditions to all or portion of the stem-loop sequence.

5. A method of selectively modifying a nucleic acid according to claim 1, wherein said substrate is a diazo substrate.

6. A method of selectively modifying a nucleic acid according to claim 1, wherein the composition is treated with a rhodium-carbenoid.

7. A method of selectively modifying a nucleic acid according to any of the proceeding claims, further comprises detecting a nucleic acid in a biological or an artificial composition, comprising:
a. treating the composition comprising the nucleic acid with a rhodium catalyst and a substrate;
b. contacting the treated nucleic acid in (a) with a probe specific for said treated nucleic acid to form a complex comprising said treated nucleic acid and said probe hybridized to said treated nucleic acid; and
c. detecting hybridization of said probe to said nucleic acid.

8. An oligonucleotide for amplifying a nucleic acid modified according to any of claims 1-6, which is identical, is complementary, or hybridizes under stringent or highly stringent conditions to the nucleic acid.

9. A method according to claim 7, further comprises amplifying a nucleic acid sequence contained in a biological or an artificial composition, comprising:
a. adding an oligonucleotide according to claim 8 to the biological composition; and
b. amplifying the nucleic acid contained in the biological sample using the oligonucleotide according to claim 8.

10. A method according to claim 9, further comprises determining the presence or absence of a genetic abnormality in a nucleic acid contained in a biological sample, comprising:
a. amplifying the nucleic acid contained in a biological or an artificial composition by a nucleic acid amplification method according to claim 9;
b. Measuring a fluorescence intensity before and after amplifying the target nucleic acid sequence in (a); and
c. Detecting the presence or absence of a genetic abnormality by comparing the fluorescence intensities measured in (b).

11. The method according to any of the proceeding claims, wherein said nucleic acid comprises an unpaired and/or mismatched nucleobase or series of unpaired and/or mismatched nucleobases.

12. A modified nucleic acid according to claim 1, and sequences complementary thereto.

13. A kit for selectively modifying a nucleic acid in a biological or an artificial composition, wherein the kit comprises (a) a rhodium catalyst (b) a substrate (c) containers suitable for containing the said rhodium catalyst, a substrate, and the composition.

14. A kit according to claim 13, wherein said catalyst further comprising a guiding motif, wherein the guiding motif is identical, is complementary, or hybridizes under stringent or highly stringent conditions to all or portion of the nucleic acid.

15. Use of a rhodium catalyst and a substrate according to any of the proceeding claims for modifying, amplifying and detecting a nucleic acid in a biological or an artificial composition.

16. A rhodium catalyst and a substrate according to any of the proceeding claims for use in generating chemical libraries of modified RNA for controlling or modulating RNA interference, or diseases of RNA misregulation, in detecting genetic abnormality in a nucleic acid, in the diagnosis, prognosis, monitoring and/or classification of cellular proliferative disorders.
